# EUROPEAN PATENT APPLICATION

(11) **EP 1 174 518 A1**
(43) Date of publication of application: **23.01.2002**
(21) Application number: 00202611.0
(22) Date of filing: 20.07.2000
(51) Int. Cl.: C12Q 1/68, C12Q 1/70

(54) **Collection of binding molecules**

(71) Applicant: Amsterdam Support Diagnostics B.V., 1105 BA Amsterdam (NL)
(72) Inventor: Loukachov, Vladimir V., 1112 RB Diemen (NL); Van Gemen, Bob, 1326 HV Almere (NL); Goudsmit, Jaap, 1075 CX Amsterdam (NL)
(74) Representative: Prins, Adrianus Willem

(57) **Abstract**

In one aspect the invention provides a collection of binding moieties for a family of nucleic acids, which family comprises members of relative high and low significance, whereby said binding moieties are selected to be capable to identify, alone or in combination essentially all members of said family of nucleic acids of relative high significance. In another aspect the invention provides methods and uses for determining or typing samples, preferably clinical samples, using a collection of binding molecules of the invention.

### Annex to the application documents-subsequently filed sequences listing

SEQUENCE LISTING

<110> Amsterdam Support Diagnostics B.V.

<120> Collection of binding molecules.

<130> P51609EP00

<140> 00202611.0
<141> 2000-07-20

<160> 543

<170> PatentIn Ver. 2.1

<210> 1
<211> 23
<212> DNA
<213> Artificial Sequence

<220>
<223> Description of Artificial Sequence: primer 3' RT

<220>
<221> misc_feature
<222> (1)..(23)

<400> 1
tctacttgtc catgcatggc ttc                                          23


<210> 2
<211> 32
<212> DNA
<213> Artificial Sequence

<220>
<223> Description of Artificial Sequence: primer 5' PROT-I

<220>
<221> misc_feature
<222> (1)..(32)

<400> 2
aggctaattt tttagggaag atctggcctt cc                                32


<210> 3
<211> 34
<212> DNA
<213> Artificial Sequence

<220>
<223> Description of Artificial Sequence: primer 3' ET21

<220>
<221> misc_feature
<222> (1)..(34)

<400> 3
agctggctac tatttctttt gctactacag gtgg                              34


<210> 4
<211> 22
<212> DNA

<213> Artificial Sequence

<220>
<223> Description of Artificial Sequence: primer RT19-new

<220>
<221> misc_feature
<222> (1)..(22)

<400> 4
cacctgtcaa cataattgga ag                                                22


<210> 5
<211> 28
<212> DNA
<213> Artificial Sequence

<220>
<223> Description of Artificial Sequence: primer 3' RT22

<220>
<221> misc_feature
<222> (1)..(28)

<400> 5
aggttaaaat cactagccat tgctctcc                                          28


<210> 6
<211> 57
<212> DNA
<213> Artificial Sequence

<220>
<223> Description of Artificial Sequence: primer 5' PROT-I-T7

<220>
<221> misc_feature
<222> (1)..(57)

<400> 6
aattctaata cgactcacta tagggaggct aattttttag ggaagatctg gccttcc    57


<210> 7
<211> 59
<212> DNA
<213> Artificial Sequence

<220>
<223> Description of Artificial Sequence: primer 3' ET21-T7

<220>
<221> misc_feature
<222> (1)..(59)

<400> 7
aattctaata cgactcacta tagggagctg gctactattt cttttgctac tacaggtgg  59


<210> 8
<211> 47
<212> DNA
<213> Artificial Sequence

<220>
<223> Description of Artificial Sequence: primer RT19-new-T7

<220>
<221> misc_feature
<222> (1)..(47)

<400> 8
aattctaata cgactcacta tagggcacct gtcaacataa ttggaag                47


<210> 9
<211> 53
<212> DNA
<213> Artificial Sequence

<220>
<223> Description of Artificial Sequence: primer 3' RT22-T7

<220>
<221> misc_feature
<222> (1)..(53)

<400> 9
aattctaata cgactcacta tagggaggtt aaaatcacta gccattgctc tcc         53


<210> 10
<211> 18
<212> DNA
<213> Artificial Sequence

<220>
<223> Description of Artificial Sequence: position 41

<220>
<221> misc_feature
<222> (1)..(18)

<400> 10
gtacagaaat ggaaaagg                                                18


<210> 11
<211> 18
<212> DNA
<213> Artificial Sequence

<220>
<223> Description of Artificial Sequence: position 41

<220>
<221> misc_feature
<222> (1)..(18)

<400> 11
gtacagagat ggaaaagg                                                18


<210> 12
<211> 18
<212> DNA
<213> Artificial Sequence

<220>
<223> Description of Artificial Sequence: position 41

<220>
<221> misc_feature
<222> (1)..(18)

<400> 12
gtgcagaaat ggaaaagg                                                    18


<210> 13
<211> 18
<212> DNA
<213> Artificial Sequence

<220>
<223> Description of Artificial Sequence: position 41

<220>
<221> misc_feature
<222> (1)..(18)

<400> 13
gtacagaaat ggaaaaag                                                    18


<210> 14
<211> 18
<212> DNA
<213> Artificial Sequence

<220>
<223> Description of Artificial Sequence: position 41

<220>
<221> misc_feature
<222> (1)..(18)

<400> 14
gtacagaaat ggagaagg                                                    18


<210> 15
<211> 18
<212> DNA
<213> Artificial Sequence

<220>
<223> Description of Artificial Sequence: position 41

<220>
<221> misc_feature
<222> (1)..(18)

<400> 15
gtacagaact ggaaaagg                                                    18


<210> 16
<211> 18
<212> DNA
<213> Artificial Sequence

<220>
<223> Description of Artificial Sequence: position 41

<220>

<221> misc_feature
<222> (1)..(18)

<400> 16
gtacagagat ggaacagg                                                    18


<210> 17
<211> 18
<212> DNA
<213> Artificial Sequence

<220>
<223> Description of Artificial Sequence: position 41

<220>
<221> misc_feature
<222> (1)..(18)

<400> 17
gcacagaaat ggaaaagg                                                    18


<210> 18
<211> 18
<212> DNA
<213> Artificial Sequence

<220>
<223> Description of Artificial Sequence: position 41

<220>
<221> misc_feature
<222> (1)..(18)

<400> 18
gtacagaatt ggaaaagg                                                    18


<210> 19
<211> 18
<212> DNA
<213> Artificial Sequence

<220>
<223> Description of Artificial Sequence: position 41

<220>
<221> misc_feature
<222> (1)..(18)

<400> 19
gtacagaaat ggaagagg                                                    18


<210> 20
<211> 18
<212> DNA
<213> Artificial Sequence

<220>
<223> Description of Artificial Sequence: position 41

<220>
<221> misc_feature
<222> (1)..(18)

<400> 20
gtacagaaat cgaaaagg                                                    18


<210> 21
<211> 18
<212> DNA
<213> Artificial Sequence

<220>
<223> Description of Artificial Sequence: position 41

<220>
<221> misc_feature
<222> (1)..(18)

<400> 21
gtacagaact ggagaagg                                                    18


<210> 22
<211> 18
<212> DNA
<213> Artificial Sequence

<220>
<223> Description of Artificial Sequence: position 41

<220>
<221> misc_feature
<222> (1)..(18)

<400> 22
gtccagaaat ggaaaagg                                                    18


<210> 23
<211> 18
<212> DNA
<213> Artificial Sequence

<220>
<223> Description of Artificial Sequence: position 41

<220>
<221> misc_feature
<222> (1)..(18)

<400> 23
gtaaagagat ggaacagg                                                    18


<210> 24
<211> 18
<212> DNA
<213> Artificial Sequence

<220>
<223> Description of Artificial Sequence: position 41

<220>
<221> misc_feature
<222> (1)..(18)

<400> 24

gtacatttat ggaagagg                                                    18

<210> 25
<211> 18
<212> DNA
<213> Artificial Sequence

<220>
<223> Description of Artificial Sequence: position 41

<220>
<221> misc_feature
<222> (1)..(18)

<400> 25
gtgcagagat ggaaaagg                                                    18

<210> 26
<211> 18
<212> DNA
<213> Artificial Sequence

<220>
<223> Description of Artificial Sequence: position 41

<220>
<221> misc_feature
<222> (1)..(18)

<400> 26
gtttagaaat ggaaaagg                                                    18

<210> 27
<211> 18
<212> DNA
<213> Artificial Sequence

<220>
<223> Description of Artificial Sequence: position 41

<220>
<221> misc_feature
<222> (1)..(18)

<400> 27
ctacagaaat ggaaaagg                                                    18

<210> 28
<211> 18
<212> DNA
<213> Artificial Sequence

<220>
<223> Description of Artificial Sequence: position 41

<220>
<221> misc_feature
<222> (1)..(18)

<400> 28
gtacatttat ggaggaag                                                    18

<210> 29
<211> 18
<212> DNA
<213> Artificial Sequence

<220>
<223> Description of Artificial Sequence: position 41

<220>
<221> misc_feature
<222> (1)..(18)

<400> 29
gtacagaact gcaacagg                                                    18


<210> 30
<211> 18
<212> DNA
<213> Artificial Sequence

<220>
<223> Description of Artificial Sequence: position 41

<220>
<221> misc_feature
<222> (1)..(18)

<400> 30
gtagagaaat ggaaaagg                                                    18


<210> 31
<211> 18
<212> DNA
<213> Artificial Sequence

<220>
<223> Description of Artificial Sequence: position 41

<220>
<221> misc_feature
<222> (1)..(18)

<400> 31
gtacagagat ggagaagg                                                    18


<210> 32
<211> 18
<212> DNA
<213> Artificial Sequence

<220>
<223> Description of Artificial Sequence: position 41

<220>
<221> misc_feature
<222> (1)..(18)

<400> 32
gtacagagct ggaaaagg                                                    18


<210> 33

<211> 18
<212> DNA
<213> Artificial Sequence

<220>
<223> Description of Artificial Sequence: position 41

<220>
<221> misc_feature
<222> (1)..(18)

<400> 33
gtacagacat ggaacagg                                                    18


<210> 34
<211> 18
<212> DNA
<213> Artificial Sequence

<220>
<223> Description of Artificial Sequence: position 41

<220>
<221> misc_feature
<222> (1)..(18)

<400> 34
gtacagagtt ggaagaag                                                    18


<210> 35
<211> 18
<212> DNA
<213> Artificial Sequence

<220>
<223> Description of Artificial Sequence: position 41

<220>
<221> misc_feature
<222> (1)..(18)

<400> 35
gtacagatat ggaaaagg                                                    18


<210> 36
<211> 18
<212> DNA
<213> Artificial Sequence

<220>
<223> Description of Artificial Sequence: position 41

<220>
<221> misc_feature
<222> (1)..(18)

<400> 36
gtacagaaat ggaaaagg                                                    18


<210> 37
<211> 18
<212> DNA

<213> Artificial Sequence

<220>
<223> Description of Artificial Sequence: position 41

<220>
<221> misc_feature
<222> (1)..(18)

<400> 37
gtacagatat ggaaaaag                                                    18


<210> 38
<211> 18
<212> DNA
<213> Artificial Sequence

<220>
<223> Description of Artificial Sequence: position 41

<220>
<221> misc_feature
<222> (1)..(18)

<400> 38
gtacagaaat ggaggagg                                                    18


<210> 39
<211> 18
<212> DNA
<213> Artificial Sequence

<220>
<223> Description of Artificial Sequence: position 41

<220>
<221> misc_feature
<222> (1)..(18)

<400> 39
gtacagaact ggagcagg                                                    18


<210> 40
<211> 18
<212> DNA
<213> Artificial Sequence

<220>
<223> Description of Artificial Sequence: position 41

<220>
<221> misc_feature
<222> (1)..(18)

<400> 40
gtgcagaact agaaaaag                                                    18


<210> 41
<211> 18
<212> DNA
<213> Artificial Sequence

<220>
<223> Description of Artificial Sequence: position 41

<220>
<221> misc_feature
<222> (1)..(18)

<400> 41
ctacagaaat gcaacagg                                                    18


<210> 42
<211> 18
<212> DNA
<213> Artificial Sequence

<220>
<223> Description of Artificial Sequence: position 41

<220>
<221> misc_feature
<222> (1)..(18)

<400> 42
gcactgaaat ggaaaaag                                                    18


<210> 43
<211> 18
<212> DNA
<213> Artificial Sequence

<220>
<223> Description of Artificial Sequence: position 41

<220>
<221> misc_feature
<222> (1)..(18)

<400> 43
gcacagaact ggagaagg                                                    18


<210> 44
<211> 18
<212> DNA
<213> Artificial Sequence

<220>
<223> Description of Artificial Sequence: position 41

<220>
<221> misc_feature
<222> (1)..(18)

<400> 44
gttcagagat ggaaaagg                                                    18


<210> 45
<211> 18
<212> DNA
<213> Artificial Sequence

<220>
<223> Description of Artificial Sequence: position 41

<220>
<221> misc_feature
<222> (1)..(18)

<400> 45
gtggagaact ggaaaagg                                                    18


<210> 46
<211> 18
<212> DNA
<213> Artificial Sequence

<220>
<223> Description of Artificial Sequence: position 41

<220>
<221> misc_feature
<222> (1)..(18)

<400> 46
gtgaagatat ggaaaagg                                                    18


<210> 47
<211> 18
<212> DNA
<213> Artificial Sequence

<220>
<223> Description of Artificial Sequence: position 41

<220>
<221> misc_feature
<222> (1)..(18)

<400> 47
gtgaagacat ggaaaagg                                                    18


<210> 48
<211> 18
<212> DNA
<213> Artificial Sequence

<220>
<223> Description of Artificial Sequence: position 41

<220>
<221> misc_feature
<222> (1)..(18)

<400> 48
gtgaagaaat ggagaagg                                                    18


<210> 49
<211> 18
<212> DNA
<213> Artificial Sequence

<220>
<223> Description of Artificial Sequence: position 41

<220>

<221> misc_feature
<222> (1)..(18)

<400> 49
gtgcatttat ggaggagg                                                    18


<210> 50
<211> 18
<212> DNA
<213> Artificial Sequence

<220>
<223> Description of Artificial Sequence: position 41

<220>
<221> misc_feature
<222> (1)..(18)

<400> 50
gtaaatttct ggaagagg                                                    18


<210> 51
<211> 18
<212> DNA
<213> Artificial Sequence

<220>
<223> Description of Artificial Sequence: position 41

<220>
<221> misc_feature
<222> (1)..(18)

<400> 51
gtgcagacat ggaaaagg                                                    18


<210> 52
<211> 18
<212> DNA
<213> Artificial Sequence

<220>
<223> Description of Artificial Sequence: position 41

<220>
<221> misc_feature
<222> (1)..(18)

<400> 52
gtaccgaaat ggaaaagg                                                    18


<210> 53
<211> 18
<212> DNA
<213> Artificial Sequence

<220>
<223> Description of Artificial Sequence: position 41

<220>
<221> misc_feature
<222> (1)..(18)

<400> 53
gtgcagaact ggaagagg                                                    18


<210> 54
<211> 18
<212> DNA
<213> Artificial Sequence

<220>
<223> Description of Artificial Sequence: position 41

<220>
<221> misc_feature
<222> (1)..(18)

<400> 54
gtgcagaact ggaaaaag                                                    18


<210> 55
<211> 18
<212> DNA
<213> Artificial Sequence

<220>
<223> Description of Artificial Sequence: position 41

<220>
<221> misc_feature
<222> (1)..(18)

<400> 55
gtgcagaact ggaaaagg                                                    18


<210> 56
<211> 18
<212> DNA
<213> Artificial Sequence

<220>
<223> Description of Artificial Sequence: position 41

<220>
<221> misc_feature
<222> (1)..(18)

<400> 56
gtgcagaaat ggaagagg                                                    18


<210> 57
<211> 18
<212> DNA
<213> Artificial Sequence

<220>
<223> Description of Artificial Sequence: position 41

<220>
<221> misc_feature
<222> (1)..(18)

<400> 57

gtgcagaaat ggaacagg                                                    18

<210> 58
<211> 18
<212> DNA
<213> Artificial Sequence

<220>
<223> Description of Artificial Sequence: position 41

<220>
<221> misc_feature
<222> (1)..(18)

<400> 58
gtctagaaat ggaaaagg                                                    18

<210> 59
<211> 18
<212> DNA
<213> Artificial Sequence

<220>
<223> Description of Artificial Sequence: position 41

<220>
<221> misc_feature
<222> (1)..(18)

<400> 59
gtcaagaaat ggaaaagg                                                    18

<210> 60
<211> 18
<212> DNA
<213> Artificial Sequence

<220>
<223> Description of Artificial Sequence: position 41

<220>
<221> misc_feature
<222> (1)..(18)

<400> 60
gtaatgaaat ggaaaagg                                                    18

<210> 61
<211> 18
<212> DNA
<213> Artificial Sequence

<220>
<223> Description of Artificial Sequence: position 41

<220>
<221> misc_feature
<222> (1)..(18)

<400> 61
gtaaagaaat ggaacagg                                                    18

<210> 62
<211> 18
<212> DNA
<213> Artificial Sequence

<220>
<223> Description of Artificial Sequence: position 41

<220>
<221> misc_feature
<222> (1)..(18)

<400> 62
gtgcagatat ggaaaagg                                                    18


<210> 63
<211> 19
<212> DNA
<213> Artificial Sequence

<220>
<223> Description of Artificial Sequence: position 62

<220>
<221> misc_feature
<222> (1)..(19)

<400> 63
cagtatttgc cataaagaa                                                   19


<210> 64
<211> 19
<212> DNA
<213> Artificial Sequence

<220>
<223> Description of Artificial Sequence: position 62

<220>
<221> misc_feature
<222> (1)..(19)

<400> 64
caatatttgc cataaagaa                                                   19


<210> 65
<211> 19
<212> DNA
<213> Artificial Sequence

<220>
<223> Description of Artificial Sequence: position 62

<220>
<221> misc_feature
<222> (1)..(19)

<400> 65
cagtatttgc tataaagaa                                                   19


<210> 66

<211> 19
<212> DNA
<213> Artificial Sequence

<220>
<223> Description of Artificial Sequence: position 62

<220>
<221> misc_feature
<222> (1)..(19)

<400> 66
cagtgtttgc cataaagaa                                                    19


<210> 67
<211> 19
<212> DNA
<213> Artificial Sequence

<220>
<223> Description of Artificial Sequence: position 62

<220>
<221> misc_feature
<222> (1)..(19)

<400> 67
caatatttgc tataaagaa                                                    19


<210> 68
<211> 19
<212> DNA
<213> Artificial Sequence

<220>
<223> Description of Artificial Sequence: position 62

<220>
<221> misc_feature
<222> (1)..(19)

<400> 68
cagtatttgc cataaaaaa                                                    19


<210> 69
<211> 19
<212> DNA
<213> Artificial Sequence

<220>
<223> Description of Artificial Sequence: position 62

<220>
<221> misc_feature
<222> (1)..(19)

<400> 69
cagtattcgc cataaagaa                                                    19


<210> 70
<211> 19
<212> DNA

<213> Artificial Sequence

<220>
<223> Description of Artificial Sequence: position 62

<220>
<221> misc_feature
<222> (1)..(19)

<400> 70
cagtatttgc cataaggaa                                                    19


<210> 71
<211> 19
<212> DNA
<213> Artificial Sequence

<220>
<223> Description of Artificial Sequence: position 62

<220>
<221> misc_feature
<222> (1)..(19)

<400> 71
cagtatttgt cataaagaa                                                    19


<210> 72
<211> 19
<212> DNA
<213> Artificial Sequence

<220>
<223> Description of Artificial Sequence: position 62

<220>
<221> misc_feature
<222> (1)..(19)

<400> 72
cagtatttgt tataaggaa                                                    19


<210> 73
<211> 19
<212> DNA
<213> Artificial Sequence

<220>
<223> Description of Artificial Sequence: position 62

<220>
<221> misc_feature
<222> (1)..(19)

<400> 73
caatatttgc aataaagaa                                                    19


<210> 74
<211> 19
<212> DNA
<213> Artificial Sequence

<220>
<223> Description of Artificial Sequence: position 62

<220>
<221> misc_feature
<222> (1)..(19)

<400> 74
cagtatttgc catacacaa                                                    19


<210> 75
<211> 19
<212> DNA
<213> Artificial Sequence

<220>
<223> Description of Artificial Sequence: position 62

<220>
<221> misc_feature
<222> (1)..(19)

<400> 75
cggtatttgc tataaagaa                                                    19


<210> 76
<211> 19
<212> DNA
<213> Artificial Sequence

<220>
<223> Description of Artificial Sequence: position 62

<220>
<221> misc_feature
<222> (1)..(19)

<400> 76
cagtatttgc cattaagaa                                                    19


<210> 77
<211> 19
<212> DNA
<213> Artificial Sequence

<220>
<223> Description of Artificial Sequence: position 62

<220>
<221> misc_feature
<222> (1)..(19)

<400> 77
cagtttttgc tataaagaa                                                    19


<210> 78
<211> 19
<212> DNA
<213> Artificial Sequence

<220>
<223> Description of Artificial Sequence: position 62

<220>
<221> misc_feature
<222> (1)..(19)

<400> 78
caatatttgc cataaaaaa                                                    19


<210> 79
<211> 19
<212> DNA
<213> Artificial Sequence

<220>
<223> Description of Artificial Sequence: position 62

<220>
<221> misc_feature
<222> (1)..(19)

<400> 79
caatatttgc cataaagga                                                    19


<210> 80
<211> 19
<212> DNA
<213> Artificial Sequence

<220>
<223> Description of Artificial Sequence: position 62

<220>
<221> misc_feature
<222> (1)..(19)

<400> 80
caatatttgc cataaggaa                                                    19


<210> 81
<211> 19
<212> DNA
<213> Artificial Sequence

<220>
<223> Description of Artificial Sequence: position 62

<220>
<221> misc_feature
<222> (1)..(19)

<400> 81
ccatatttgc cataaagaa                                                    19


<210> 82
<211> 19
<212> DNA
<213> Artificial Sequence

<220>
<223> Description of Artificial Sequence: position 62

<220>

<221> misc_feature
<222> (1)..(19)

<400> 82
ccatatttgc cataaggaa                                                    19


<210> 83
<211> 19
<212> DNA
<213> Artificial Sequence

<220>
<223> Description of Artificial Sequence: position 62

<220>
<221> misc_feature
<222> (1)..(19)

<400> 83
cggtatttgc cataaagaa                                                    19


<210> 84
<211> 19
<212> DNA
<213> Artificial Sequence

<220>
<223> Description of Artificial Sequence: position 62

<220>
<221> misc_feature
<222> (1)..(19)

<400> 84
cggtatttgc aataaagaa                                                    19


<210> 85
<211> 19
<212> DNA
<213> Artificial Sequence

<220>
<223> Description of Artificial Sequence: position 62

<220>
<221> misc_feature
<222> (1)..(19)

<400> 85
cagtgttcgc cataaaaaa                                                    19


<210> 86
<211> 19
<212> DNA
<213> Artificial Sequence

<220>
<223> Description of Artificial Sequence: position 62

<220>
<221> misc_feature
<222> (1)..(19)

<400> 86
cagtgtttgc catacacaa                                                    19


<210> 87
<211> 19
<212> DNA
<213> Artificial Sequence

<220>
<223> Description of Artificial Sequence: position 62

<220>
<221> misc_feature
<222> (1)..(19)

<400> 87
cagtgtttgc tataaagaa                                                    19


<210> 88
<211> 19
<212> DNA
<213> Artificial Sequence

<220>
<223> Description of Artificial Sequence: position 62

<220>
<221> misc_feature
<222> (1)..(19)

<400> 88
cagtatttgc catatacaa                                                    19


<210> 89
<211> 19
<212> DNA
<213> Artificial Sequence

<220>
<223> Description of Artificial Sequence: position 62

<220>
<221> misc_feature
<222> (1)..(19)

<400> 89
cagtatttgc tattaagaa                                                    19


<210> 90
<211> 19
<212> DNA
<213> Artificial Sequence

<220>
<223> Description of Artificial Sequence: position 65

<220>
<221> misc_feature
<222> (1)..(19)

<400> 90

ccataaagaa aaaagacag 19

<210> 91
<211> 19
<212> DNA
<213> Artificial Sequence

<220>
<223> Description of Artificial Sequence: position 65

<220>
<221> misc_feature
<222> (1)..(19)

<400> 91
ccataaagaa aaagaacag 19

<210> 92
<211> 19
<212> DNA
<213> Artificial Sequence

<220>
<223> Description of Artificial Sequence: position 65

<220>
<221> misc_feature
<222> (1)..(19)

<400> 92
ccataaagaa aaaggacag 19

<210> 93
<211> 19
<212> DNA
<213> Artificial Sequence

<220>
<223> Description of Artificial Sequence: position 65

<220>
<221> misc_feature
<222> (1)..(19)

<400> 93
ctataaagaa aaaagacag 19

<210> 94
<211> 19
<212> DNA
<213> Artificial Sequence

<220>
<223> Description of Artificial Sequence: position 65

<220>
<221> misc_feature
<222> (1)..(19)

<400> 94
ccataaagaa aaaagatag 19

<210> 95
<211> 19
<212> DNA
<213> Artificial Sequence

<220>
<223> Description of Artificial Sequence: position 65

<220>
<221> misc_feature
<222> (1)..(19)

<400> 95
ccataaagaa gaaagacag                                                    19


<210> 96
<211> 19
<212> DNA
<213> Artificial Sequence

<220>
<223> Description of Artificial Sequence: position 65

<220>
<221> misc_feature
<222> (1)..(19)

<400> 96
ccataaagaa aaaagatgg                                                    19


<210> 97
<211> 19
<212> DNA
<213> Artificial Sequence

<220>
<223> Description of Artificial Sequence: position 65

<220>
<221> misc_feature
<222> (1)..(19)

<400> 97
ccataaagaa aaaaaacag                                                    19


<210> 98
<211> 19
<212> DNA
<213> Artificial Sequence

<220>
<223> Description of Artificial Sequence: position 65

<220>
<221> misc_feature
<222> (1)..(19)

<400> 98
ccataaagaa gaaaaacag                                                    19


<210> 99

<211> 19
<212> DNA
<213> Artificial Sequence

<220>
<223> Description of Artificial Sequence: position 65

<220>
<221> misc_feature
<222> (1)..(19)

<400> 99
ctataaagaa aaaaaatag                                                    19


<210> 100
<211> 19
<212> DNA
<213> Artificial Sequence

<220>
<223> Description of Artificial Sequence: position 65

<220>
<221> misc_feature
<222> (1)..(19)

<400> 100
ctataaagaa aaaggacag                                                    19


<210> 101
<211> 19
<212> DNA
<213> Artificial Sequence

<220>
<223> Description of Artificial Sequence: position 65

<220>
<221> misc_feature
<222> (1)..(19)

<400> 101
ctataaagaa aaagaacag                                                    19


<210> 102
<211> 19
<212> DNA
<213> Artificial Sequence

<220>
<223> Description of Artificial Sequence: position 65

<220>
<221> misc_feature
<222> (1)..(19)

<400> 102
ctataaagaa aaaagatag                                                    19


<210> 103
<211> 19
<212> DNA

<213> Artificial Sequence

<220>
<223> Description of Artificial Sequence: position 65

<220>
<221> misc_feature
<222> (1)..(19)

<400> 103
ccataaagaa aaaaggcag                                                    19


<210> 104
<211> 19
<212> DNA
<213> Artificial Sequence

<220>
<223> Description of Artificial Sequence: position 65

<220>
<221> misc_feature
<222> (1)..(19)

<400> 104
ccataaagaa aaaggatgg                                                    19


<210> 105
<211> 19
<212> DNA
<213> Artificial Sequence

<220>
<223> Description of Artificial Sequence: position 65

<220>
<221> misc_feature
<222> (1)..(19)

<400> 105
tcataaagaa aaaagacag                                                    19


<210> 106
<211> 19
<212> DNA
<213> Artificial Sequence

<220>
<223> Description of Artificial Sequence: position 65

<220>
<221> misc_feature
<222> (1)..(19)

<400> 106
ttataaggaa aaaagaaag                                                    19


<210> 107
<211> 19
<212> DNA
<213> Artificial Sequence

<220>
<223> Description of Artificial Sequence: position 65

<220>
<221> misc_feature
<222> (1)..(19)

<400> 107
ccataaaaaa gaaagacag                                                    19


<210> 108
<211> 19
<212> DNA
<213> Artificial Sequence

<220>
<223> Description of Artificial Sequence: position 65

<220>
<221> misc_feature
<222> (1)..(19)

<400> 108
ccatacacaa gaaaaacag                                                    19


<210> 109
<211> 19
<212> DNA
<213> Artificial Sequence

<220>
<223> Description of Artificial Sequence: position 65

<220>
<221> misc_feature
<222> (1)..(19)

<400> 109
ccataaggaa aaaagacag                                                    19


<210> 110
<211> 19
<212> DNA
<213> Artificial Sequence

<220>
<223> Description of Artificial Sequence: position 65

<220>
<221> misc_feature
<222> (1)..(19)

<400> 110
ccataaagaa aaagaatag                                                    19


<210> 111
<211> 19
<212> DNA
<213> Artificial Sequence

<220>
<223> Description of Artificial Sequence: position 65

<220>
<221> misc_feature
<222> (1)..(19)

<400> 111
ccattaagaa aaaagacag                                                    19


<210> 112
<211> 19
<212> DNA
<213> Artificial Sequence

<220>
<223> Description of Artificial Sequence: position 65

<220>
<221> misc_feature
<222> (1)..(19)

<400> 112
ccataaagaa aaaagaaag                                                    19


<210> 113
<211> 19
<212> DNA
<213> Artificial Sequence

<220>
<223> Description of Artificial Sequence: position 65

<220>
<221> misc_feature
<222> (1)..(19)

<400> 113
ccataaaaaa aaaagacag                                                    19


<210> 114
<211> 19
<212> DNA
<213> Artificial Sequence

<220>
<223> Description of Artificial Sequence: position 65

<220>
<221> misc_feature
<222> (1)..(19)

<400> 114
ccataaagaa aaaagaagg                                                    19


<210> 115
<211> 19
<212> DNA
<213> Artificial Sequence

<220>
<223> Description of Artificial Sequence: position 65

<220>

<221> misc_feature
<222> (1)..(19)

<400> 115
caataaagaa aaaggacag                                                    19


<210> 116
<211> 19
<212> DNA
<213> Artificial Sequence

<220>
<223> Description of Artificial Sequence: position 65

<220>
<221> misc_feature
<222> (1)..(19)

<400> 116
caataaagaa gaaagacag                                                    19


<210> 117
<211> 19
<212> DNA
<213> Artificial Sequence

<220>
<223> Description of Artificial Sequence: position 65

<220>
<221> misc_feature
<222> (1)..(19)

<400> 117
ccataaaaaa gaaaaacag                                                    19


<210> 118
<211> 19
<212> DNA
<213> Artificial Sequence

<220>
<223> Description of Artificial Sequence: position 65

<220>
<221> misc_feature
<222> (1)..(19)

<400> 118
ccataaaaaa gaaggacag                                                    19


<210> 119
<211> 19
<212> DNA
<213> Artificial Sequence

<220>
<223> Description of Artificial Sequence: position 65

<220>
<221> misc_feature
<222> (1)..(19)

<400> 119
ccataaaaaa gaagaacag                                                    19


<210> 120
<211> 19
<212> DNA
<213> Artificial Sequence

<220>
<223> Description of Artificial Sequence: position 65

<220>
<221> misc_feature
<222> (1)..(19)

<400> 120
caataaagaa gaaagatag                                                    19


<210> 121
<211> 19
<212> DNA
<213> Artificial Sequence

<220>
<223> Description of Artificial Sequence: position 65

<220>
<221> misc_feature
<222> (1)..(19)

<400> 121
ccatacacaa aaaaggcag                                                    19


<210> 122
<211> 19
<212> DNA
<213> Artificial Sequence

<220>
<223> Description of Artificial Sequence: position 65

<220>
<221> misc_feature
<222> (1)..(19)

<400> 122
ccataaagaa aaaagagag                                                    19


<210> 123
<211> 19
<212> DNA
<213> Artificial Sequence

<220>
<223> Description of Artificial Sequence: position 65

<220>
<221> misc_feature
<222> (1)..(19)

<400> 123

ccataaagaa aagagacag                                                    19

<210> 124
<211> 19
<212> DNA
<213> Artificial Sequence

<220>
<223> Description of Artificial Sequence: position 65

<220>
<221> misc_feature
<222> (1)..(19)

<400> 124
ccataaagag aaaagacag                                                    19

<210> 125
<211> 19
<212> DNA
<213> Artificial Sequence

<220>
<223> Description of Artificial Sequence: position 65

<220>
<221> misc_feature
<222> (1)..(19)

<400> 125
ccataaagga aaaaaacag                                                    19

<210> 126
<211> 19
<212> DNA
<213> Artificial Sequence

<220>
<223> Description of Artificial Sequence: position 65

<220>
<221> misc_feature
<222> (1)..(19)

<400> 126
ccataaagaa gaaaagcag                                                    19

<210> 127
<211> 19
<212> DNA
<213> Artificial Sequence

<220>
<223> Description of Artificial Sequence: position 65

<220>
<221> misc_feature
<222> (1)..(19)

<400> 127
ccataaggaa aaaggacag                                                    19

<210> 128
<211> 19
<212> DNA
<213> Artificial Sequence

<220>
<223> Description of Artificial Sequence: position 65

<220>
<221> misc_feature
<222> (1)..(19)

<400> 128
ccataaggaa aaagaacag                                                    19


<210> 129
<211> 19
<212> DNA
<213> Artificial Sequence

<220>
<223> Description of Artificial Sequence: position 65

<220>
<221> misc_feature
<222> (1)..(19)

<400> 129
ccataaggaa gaaaaacag                                                    19


<210> 130
<211> 19
<212> DNA
<213> Artificial Sequence

<220>
<223> Description of Artificial Sequence: position 65

<220>
<221> misc_feature
<222> (1)..(19)

<400> 130
ccataaagaa aaaggatag                                                    19


<210> 131
<211> 19
<212> DNA
<213> Artificial Sequence

<220>
<223> Description of Artificial Sequence: position 65

<220>
<221> misc_feature
<222> (1)..(19)

<400> 131
ccatatacaa gaaaaacag                                                    19


<210> 132

<211> 19
<212> DNA
<213> Artificial Sequence

<220>
<223> Description of Artificial Sequence: position 65

<220>
<221> misc_feature
<222> (1)..(19)

<400> 132
ctataaagaa gaaaaaaag                                                    19


<210> 133
<211> 19
<212> DNA
<213> Artificial Sequence

<220>
<223> Description of Artificial Sequence: position 65

<220>
<221> misc_feature
<222> (1)..(19)

<400> 133
ctataaagaa aaagaatag                                                    19


<210> 134
<211> 19
<212> DNA
<213> Artificial Sequence

<220>
<223> Description of Artificial Sequence: position 65

<220>
<221> misc_feature
<222> (1)..(19)

<400> 134
ctattaagaa aaaagacag                                                    19


<210> 135
<211> 19
<212> DNA
<213> Artificial Sequence

<220>
<223> Description of Artificial Sequence: position 67

<220>
<221> misc_feature
<222> (1)..(19)

<400> 135
agaaaaaaga cagtactaa                                                    19


<210> 136
<211> 19
<212> DNA

<213> Artificial Sequence

<220>
<223> Description of Artificial Sequence: position 67

<220>
<221> misc_feature
<222> (1)..(19)

<400> 136
agaaaaagga cagtactaa                                                    19


<210> 137
<211> 19
<212> DNA
<213> Artificial Sequence

<220>
<223> Description of Artificial Sequence: position 67

<220>
<221> misc_feature
<222> (1)..(19)

<400> 137
agaaaaaaga tagtactaa                                                    19


<210> 138
<211> 19
<212> DNA
<213> Artificial Sequence

<220>
<223> Description of Artificial Sequence: position 67

<220>
<221> misc_feature
<222> (1)..(19)

<400> 138
agaagaaaga cagtactaa                                                    19


<210> 139
<211> 19
<212> DNA
<213> Artificial Sequence

<220>
<223> Description of Artificial Sequence: position 67

<220>
<221> misc_feature
<222> (1)..(19)

<400> 139
agaaaaagaa cagtactaa                                                    19


<210> 140
<211> 19
<212> DNA
<213> Artificial Sequence

<220>
<223> Description of Artificial Sequence: position 67

<220>
<221> misc_feature
<222> (1)..(19)

<400> 140
agaaaaaaga tggtactaa                                                    19


<210> 141
<211> 19
<212> DNA
<213> Artificial Sequence

<220>
<223> Description of Artificial Sequence: position 67

<220>
<221> misc_feature
<222> (1)..(19)

<400> 141
agaaaaaaga cagcactaa                                                    19


<210> 142
<211> 19
<212> DNA
<213> Artificial Sequence

<220>
<223> Description of Artificial Sequence: position 67

<220>
<221> misc_feature
<222> (1)..(19)

<400> 142
agaaaaaaga cagtaataa                                                    19


<210> 143
<211> 19
<212> DNA
<213> Artificial Sequence

<220>
<223> Description of Artificial Sequence: position 67

<220>
<221> misc_feature
<222> (1)..(19)

<400> 143
agaaaaagaa cagtgataa                                                    19


<210> 144
<211> 19
<212> DNA
<213> Artificial Sequence

<220>
<223> Description of Artificial Sequence: position 67

<220>
<221> misc_feature
<222> (1)..(19)

<400> 144
agaaaaaaga cagtgataa                                                    19


<210> 145
<211> 19
<212> DNA
<213> Artificial Sequence

<220>
<223> Description of Artificial Sequence: position 67

<220>
<221> misc_feature
<222> (1)..(19)

<400> 145
aaaagaaaga cagtactaa                                                    19


<210> 146
<211> 19
<212> DNA
<213> Artificial Sequence

<220>
<223> Description of Artificial Sequence: position 67

<220>
<221> misc_feature
<222> (1)..(19)

<400> 146
agaaaaaaga cagtaccaa                                                    19


<210> 147
<211> 19
<212> DNA
<213> Artificial Sequence

<220>
<223> Description of Artificial Sequence: position 67

<220>
<221> misc_feature
<222> (1)..(19)

<400> 147
agaaaaaaga cagtagtaa                                                    19


<210> 148
<211> 19
<212> DNA
<213> Artificial Sequence

<220>
<223> Description of Artificial Sequence: position 67

<220>

<221> misc_feature
<222> (1)..(19)

<400> 148
ggaaaaaaga cagtactaa                                                    19


<210> 149
<211> 19
<212> DNA
<213> Artificial Sequence

<220>
<223> Description of Artificial Sequence: position 67

<220>
<221> misc_feature
<222> (1)..(19)

<400> 149
agaagaaaaa cagtactaa                                                    19


<210> 150
<211> 19
<212> DNA
<213> Artificial Sequence

<220>
<223> Description of Artificial Sequence: position 67

<220>
<221> misc_feature
<222> (1)..(19)

<400> 150
ggaaaaaaga aagtggtaa                                                    19


<210> 151
<211> 19
<212> DNA
<213> Artificial Sequence

<220>
<223> Description of Artificial Sequence: position 67

<220>
<221> misc_feature
<222> (1)..(19)

<400> 151
agaaaaagga cagcactaa                                                    19


<210> 152
<211> 19
<212> DNA
<213> Artificial Sequence

<220>
<223> Description of Artificial Sequence: position 67

<220>
<221> misc_feature
<222> (1)..(19)

<400> 152
agaaaaaaga cagttctaa                                                    19


<210> 153
<211> 19
<212> DNA
<213> Artificial Sequence

<220>
<223> Description of Artificial Sequence: position 69

<220>
<221> misc_feature
<222> (1)..(19)

<400> 153
aagacagtac taaatggag                                                    19


<210> 154
<211> 19
<212> DNA
<213> Artificial Sequence

<220>
<223> Description of Artificial Sequence: position 69

<220>
<221> misc_feature
<222> (1)..(19)

<400> 154
aggacagtac taaatggag                                                    19


<210> 155
<211> 19
<212> DNA
<213> Artificial Sequence

<220>
<223> Description of Artificial Sequence: position 69

<220>
<221> misc_feature
<222> (1)..(19)

<400> 155
aagatagtac taaatggag                                                    19


<210> 156
<211> 19
<212> DNA
<213> Artificial Sequence

<220>
<223> Description of Artificial Sequence: position 69

<220>
<221> misc_feature
<222> (1)..(19)

<400> 156

agaacagtac tagatggag 19

<210> 157
<211> 19
<212> DNA
<213> Artificial Sequence

<220>
<223> Description of Artificial Sequence: position 69

<220>
<221> misc_feature
<222> (1)..(19)

<400> 157
agaacagtac taaatggag 19

<210> 158
<211> 19
<212> DNA
<213> Artificial Sequence

<220>
<223> Description of Artificial Sequence: position 69

<220>
<221> misc_feature
<222> (1)..(19)

<400> 158
aagatggtac taaatggag 19

<210> 159
<211> 19
<212> DNA
<213> Artificial Sequence

<220>
<223> Description of Artificial Sequence: position 69

<220>
<221> misc_feature
<222> (1)..(19)

<400> 159
aaaacagtac tagatggag 19

<210> 160
<211> 19
<212> DNA
<213> Artificial Sequence

<220>
<223> Description of Artificial Sequence: position 69

<220>
<221> misc_feature
<222> (1)..(19)

<400> 160
aagacagcac taaatggag 19

<210> 161
<211> 19
<212> DNA
<213> Artificial Sequence

<220>
<223> Description of Artificial Sequence: position 69

<220>
<221> misc_feature
<222> (1)..(19)

<400> 161
aagacagtac taagtggag                                                    19


<210> 162
<211> 19
<212> DNA
<213> Artificial Sequence

<220>
<223> Description of Artificial Sequence: position 69

<220>
<221> misc_feature
<222> (1)..(19)

<400> 162
agaacagtaa taggtggag                                                    19


<210> 163
<211> 19
<212> DNA
<213> Artificial Sequence

<220>
<223> Description of Artificial Sequence: position 69

<220>
<221> misc_feature
<222> (1)..(19)

<400> 163
aaaatagtac tagatggag                                                    19


<210> 164
<211> 19
<212> DNA
<213> Artificial Sequence

<220>
<223> Description of Artificial Sequence: position 69

<220>
<221> misc_feature
<222> (1)..(19)

<400> 164
aagacagtac tagatggag                                                    19


<210> 165

<211> 19
<212> DNA
<213> Artificial Sequence

<220>
<223> Description of Artificial Sequence: position 69

<220>
<221> misc_feature
<222> (1)..(19)

<400> 165
aagacagtaa taaatggag                                                    19


<210> 166
<211> 19
<212> DNA
<213> Artificial Sequence

<220>
<223> Description of Artificial Sequence: position 69

<220>
<221> misc_feature
<222> (1)..(19)

<400> 166
aaaacagtac taaatggag                                                    19


<210> 167
<211> 19
<212> DNA
<213> Artificial Sequence

<220>
<223> Description of Artificial Sequence: position 69

<220>
<221> misc_feature
<222> (1)..(19)

<400> 167
agaacagtga taaatggag                                                    19


<210> 168
<211> 19
<212> DNA
<213> Artificial Sequence

<220>
<223> Description of Artificial Sequence: position 69

<220>
<221> misc_feature
<222> (1)..(19)

<400> 168
aagacagtga taaatggag                                                    19


<210> 169
<211> 19
<212> DNA

<213> Artificial Sequence

<220>
<223> Description of Artificial Sequence: position 69

<220>
<221> misc_feature
<222> (1)..(19)

<400> 169
agaacagtga tagatggag                                                    19


<210> 170
<211> 19
<212> DNA
<213> Artificial Sequence

<220>
<223> Description of Artificial Sequence: position 69

<220>
<221> misc_feature
<222> (1)..(19)

<400> 170
aggatggtac taaatggag                                                    19


<210> 171
<211> 19
<212> DNA
<213> Artificial Sequence

<220>
<223> Description of Artificial Sequence: position 69

<220>
<221> misc_feature
<222> (1)..(19)

<400> 171
aaaacagtaa tagatggag                                                    19


<210> 172
<211> 19
<212> DNA
<213> Artificial Sequence

<220>
<223> Description of Artificial Sequence: position 69

<220>
<221> misc_feature
<222> (1)..(19)

<400> 172
aaaacagcac tagatggag                                                    19


<210> 173
<211> 19
<212> DNA
<213> Artificial Sequence

<220>
<223> Description of Artificial Sequence: position 69

<220>
<221> misc_feature
<222> (1)..(19)

<400> 173
aagacagtaa tagatggag                                                    19


<210> 174
<211> 19
<212> DNA
<213> Artificial Sequence

<220>
<223> Description of Artificial Sequence: position 69

<220>
<221> misc_feature
<222> (1)..(19)

<400> 174
aaaacagtga taaatggag                                                    19


<210> 175
<211> 19
<212> DNA
<213> Artificial Sequence

<220>
<223> Description of Artificial Sequence: position 69

<220>
<221> misc_feature
<222> (1)..(19)

<400> 175
aagaaagtgg taaatggag                                                    19


<210> 176
<211> 19
<212> DNA
<213> Artificial Sequence

<220>
<223> Description of Artificial Sequence: position 69

<220>
<221> misc_feature
<222> (1)..(19)

<400> 176
aagacagtac caaatggag                                                    19


<210> 177
<211> 19
<212> DNA
<213> Artificial Sequence

<220>
<223> Description of Artificial Sequence: position 69

<220>
<221> misc_feature
<222> (1)..(19)

<400> 177
aagacagtag taaatggag                                                    19


<210> 178
<211> 19
<212> DNA
<213> Artificial Sequence

<220>
<223> Description of Artificial Sequence: position 69

<220>
<221> misc_feature
<222> (1)..(19)

<400> 178
agaacagtac taagtggag                                                    19


<210> 179
<211> 19
<212> DNA
<213> Artificial Sequence

<220>
<223> Description of Artificial Sequence: position 69

<220>
<221> misc_feature
<222> (1)..(19)

<400> 179
agaatagtac tagatggag                                                    19


<210> 180
<211> 19
<212> DNA
<213> Artificial Sequence

<220>
<223> Description of Artificial Sequence: position 69

<220>
<221> misc_feature
<222> (1)..(19)

<400> 180
aagacagtac tagctggag                                                    19


<210> 181
<211> 19
<212> DNA
<213> Artificial Sequence

<220>
<223> Description of Artificial Sequence: position 69

<220>

<221> misc_feature
<222> (1)..(19)

<400> 181
aggacagcac taaatggag                                                    19


<210> 182
<211> 19
<212> DNA
<213> Artificial Sequence

<220>
<223> Description of Artificial Sequence: position 69

<220>
<221> misc_feature
<222> (1)..(19)

<400> 182
aggacagtac taagtggag                                                    19


<210> 183
<211> 19
<212> DNA
<213> Artificial Sequence

<220>
<223> Description of Artificial Sequence: position 69

<220>
<221> misc_feature
<222> (1)..(19)

<400> 183
aagacagttc taaatggag                                                    19


<210> 184
<211> 19
<212> DNA
<213> Artificial Sequence

<220>
<223> Description of Artificial Sequence: position 70

<220>
<221> misc_feature
<222> (1)..(19)

<400> 184
acagtactaa atggagaaa                                                    19


<210> 185
<211> 19
<212> DNA
<213> Artificial Sequence

<220>
<223> Description of Artificial Sequence: position 70

<220>
<221> misc_feature
<222> (1)..(19)

<400> 185
acagtactag atggagaaa                                                    19


<210> 186
<211> 19
<212> DNA
<213> Artificial Sequence

<220>
<223> Description of Artificial Sequence: position 70

<220>
<221> misc_feature
<222> (1)..(19)

<400> 186
atagtactaa atggagaaa                                                    19


<210> 187
<211> 19
<212> DNA
<213> Artificial Sequence

<220>
<223> Description of Artificial Sequence: position 70

<220>
<221> misc_feature
<222> (1)..(19)

<400> 187
atggtactaa atggagaaa                                                    19


<210> 188
<211> 19
<212> DNA
<213> Artificial Sequence

<220>
<223> Description of Artificial Sequence: position 70

<220>
<221> misc_feature
<222> (1)..(19)

<400> 188
acagtactaa gtggagaaa                                                    19


<210> 189
<211> 19
<212> DNA
<213> Artificial Sequence

<220>
<223> Description of Artificial Sequence: position 70

<220>
<221> misc_feature
<222> (1)..(19)

<400> 189

acagtgataa atggagaaa                                                    19

<210> 190
<211> 19
<212> DNA
<213> Artificial Sequence

<220>
<223> Description of Artificial Sequence: position 70

<220>
<221> misc_feature
<222> (1)..(19)

<400> 190
acagcactaa atggagaaa                                                    19

<210> 191
<211> 19
<212> DNA
<213> Artificial Sequence

<220>
<223> Description of Artificial Sequence: position 70

<220>
<221> misc_feature
<222> (1)..(19)

<400> 191
atagtactag atggagaaa                                                    19

<210> 192
<211> 19
<212> DNA
<213> Artificial Sequence

<220>
<223> Description of Artificial Sequence: position 70

<220>
<221> misc_feature
<222> (1)..(19)

<400> 192
acagtaatag gtggagaaa                                                    19

<210> 193
<211> 19
<212> DNA
<213> Artificial Sequence

<220>
<223> Description of Artificial Sequence: position 70

<220>
<221> misc_feature
<222> (1)..(19)

<400> 193
acagtaataa atggagaaa                                                    19

<210> 194
<211> 19
<212> DNA
<213> Artificial Sequence

<220>
<223> Description of Artificial Sequence: position 70

<220>
<221> misc_feature
<222> (1)..(19)

<400> 194
acagtaatag atggagaaa                                                    19


<210> 195
<211> 19
<212> DNA
<213> Artificial Sequence

<220>
<223> Description of Artificial Sequence: position 70

<220>
<221> misc_feature
<222> (1)..(19)

<400> 195
acagcactaa atggaggaa                                                    19


<210> 196
<211> 19
<212> DNA
<213> Artificial Sequence

<220>
<223> Description of Artificial Sequence: position 70

<220>
<221> misc_feature
<222> (1)..(19)

<400> 196
acagcactag atggagaaa                                                    19


<210> 197
<211> 19
<212> DNA
<213> Artificial Sequence

<220>
<223> Description of Artificial Sequence: position 70

<220>
<221> misc_feature
<222> (1)..(19)

<400> 197
acagtactaa atggaggaa                                                    19


<210> 198

<211> 19
<212> DNA
<213> Artificial Sequence

<220>
<223> Description of Artificial Sequence: position 70

<220>
<221> misc_feature
<222> (1)..(19)

<400> 198
acagtgatag atggagaaa                                                    19


<210> 199
<211> 19
<212> DNA
<213> Artificial Sequence

<220>
<223> Description of Artificial Sequence: position 70

<220>
<221> misc_feature
<222> (1)..(19)

<400> 199
aaagtggtaa atggagaaa                                                    19


<210> 200
<211> 19
<212> DNA
<213> Artificial Sequence

<220>
<223> Description of Artificial Sequence: position 70

<220>
<221> misc_feature
<222> (1)..(19)

<400> 200
acagtaccaa atggagaaa                                                    19


<210> 201
<211> 19
<212> DNA
<213> Artificial Sequence

<220>
<223> Description of Artificial Sequence: position 70

<220>
<221> misc_feature
<222> (1)..(19)

<400> 201
acagtaccag atggagaaa                                                    19


<210> 202
<211> 19
<212> DNA

<213> Artificial Sequence

<220>
<223> Description of Artificial Sequence: position 70

<220>
<221> misc_feature
<222> (1)..(19)

<400> 202
acagtagtaa atggagaaa                                                    19


<210> 203
<211> 19
<212> DNA
<213> Artificial Sequence

<220>
<223> Description of Artificial Sequence: position 70

<220>
<221> misc_feature
<222> (1)..(19)

<400> 203
acagtactag ctggagaaa                                                    19


<210> 204
<211> 19
<212> DNA
<213> Artificial Sequence

<220>
<223> Description of Artificial Sequence: position 70

<220>
<221> misc_feature
<222> (1)..(19)

<400> 204
acagtactag gtggagaaa                                                    19


<210> 205
<211> 19
<212> DNA
<213> Artificial Sequence

<220>
<223> Description of Artificial Sequence: position 70

<220>
<221> misc_feature
<222> (1)..(19)

<400> 205
acagttctaa atggagaaa                                                    19


<210> 206
<211> 19
<212> DNA
<213> Artificial Sequence

<220>
<223> Description of Artificial Sequence: position 74

<220>
<221> misc_feature
<222> (1)..(19)

<400> 206
ggagaaaatt agtagattt                                                    19


<210> 207
<211> 19
<212> DNA
<213> Artificial Sequence

<220>
<223> Description of Artificial Sequence: position 74

<220>
<221> misc_feature
<222> (1)..(19)

<400> 207
ggagaaaatt agtagactt                                                    19


<210> 208
<211> 19
<212> DNA
<213> Artificial Sequence

<220>
<223> Description of Artificial Sequence: position 74

<220>
<221> misc_feature
<222> (1)..(19)

<400> 208
ggagaaagtt agtagattt                                                    19


<210> 209
<211> 19
<212> DNA
<213> Artificial Sequence

<220>
<223> Description of Artificial Sequence: position 74

<220>
<221> misc_feature
<222> (1)..(19)

<400> 209
ggaggaaatt agtagattt                                                    19


<210> 210
<211> 19
<212> DNA
<213> Artificial Sequence

<220>
<223> Description of Artificial Sequence: position 74

<220>
<221> misc_feature
<222> (1)..(19)

<400> 210
ggagaaaatt ggtagattt                                                    19


<210> 211
<211> 19
<212> DNA
<213> Artificial Sequence

<220>
<223> Description of Artificial Sequence: position 74

<220>
<221> misc_feature
<222> (1)..(19)

<400> 211
ggagaaaatt aatggattt                                                    19


<210> 212
<211> 19
<212> DNA
<213> Artificial Sequence

<220>
<223> Description of Artificial Sequence: position 74

<220>
<221> misc_feature
<222> (1)..(19)

<400> 212
ggagaaaatt agtggattt                                                    19


<210> 213
<211> 19
<212> DNA
<213> Artificial Sequence

<220>
<223> Description of Artificial Sequence: position 74

<220>
<221> misc_feature
<222> (1)..(19)

<400> 213
ggagaaaact agtagattt                                                    19


<210> 214
<211> 19
<212> DNA
<213> Artificial Sequence

<220>
<223> Description of Artificial Sequence: position 74

<220>

<221> misc_feature
<222> (1)..(19)

<400> 214
ggagaaaagt agtagattt                                                    19


<210> 215
<211> 19
<212> DNA
<213> Artificial Sequence

<220>
<223> Description of Artificial Sequence: position 74

<220>
<221> misc_feature
<222> (1)..(19)

<400> 215
ggagagaatt agtagattt                                                    19


<210> 216
<211> 19
<212> DNA
<213> Artificial Sequence

<220>
<223> Description of Artificial Sequence: position 75

<220>
<221> misc_feature
<222> (1)..(19)

<400> 216
gaaaattagt agatttcag                                                    19


<210> 217
<211> 19
<212> DNA
<213> Artificial Sequence

<220>
<223> Description of Artificial Sequence: position 75

<220>
<221> misc_feature
<222> (1)..(19)

<400> 217
gaaaattagt agacttcag                                                    19


<210> 218
<211> 19
<212> DNA
<213> Artificial Sequence

<220>
<223> Description of Artificial Sequence: position 75

<220>
<221> misc_feature
<222> (1)..(19)

<400> 218
gaaaattagt agattttag                                                    19


<210> 219
<211> 19
<212> DNA
<213> Artificial Sequence

<220>
<223> Description of Artificial Sequence: position 75

<220>
<221> misc_feature
<222> (1)..(19)

<400> 219
gaaaattggt agatttcag                                                    19


<210> 220
<211> 19
<212> DNA
<213> Artificial Sequence

<220>
<223> Description of Artificial Sequence: position 75

<220>
<221> misc_feature
<222> (1)..(19)

<400> 220
ggaaattagt agatttcag                                                    19


<210> 221
<211> 19
<212> DNA
<213> Artificial Sequence

<220>
<223> Description of Artificial Sequence: position 75

<220>
<221> misc_feature
<222> (1)..(19)

<400> 221
gaaaattaat ggatttcag                                                    19


<210> 222
<211> 19
<212> DNA
<213> Artificial Sequence

<220>
<223> Description of Artificial Sequence: position 75

<220>
<221> misc_feature
<222> (1)..(19)

<400> 222

gaaaattagt ggatttcag                                                    19

<210> 223
<211> 19
<212> DNA
<213> Artificial Sequence

<220>
<223> Description of Artificial Sequence: position 75

<220>
<221> misc_feature
<222> (1)..(19)

<400> 223
gaaagttagt agatttcag                                                    19

<210> 224
<211> 19
<212> DNA
<213> Artificial Sequence

<220>
<223> Description of Artificial Sequence: position 75

<220>
<221> misc_feature
<222> (1)..(19)

<400> 224
gaaaactagt agatttcag                                                    19

<210> 225
<211> 19
<212> DNA
<213> Artificial Sequence

<220>
<223> Description of Artificial Sequence: position 75

<220>
<221> misc_feature
<222> (1)..(19)

<400> 225
gaaaagtagt agatttcag                                                    19

<210> 226
<211> 19
<212> DNA
<213> Artificial Sequence

<220>
<223> Description of Artificial Sequence: position 75

<220>
<221> misc_feature
<222> (1)..(19)

<400> 226
gaaagttagt agattttag                                                    19

<210> 227
<211> 19
<212> DNA
<213> Artificial Sequence

<220>
<223> Description of Artificial Sequence: position 77

<220>
<221> misc_feature
<222> (1)..(19)

<400> 227
tagtagattt cagagaact                                                    19


<210> 228
<211> 19
<212> DNA
<213> Artificial Sequence

<220>
<223> Description of Artificial Sequence: position 77

<220>
<221> misc_feature
<222> (1)..(19)

<400> 228
tagtagattt cagggaact                                                    19


<210> 229
<211> 19
<212> DNA
<213> Artificial Sequence

<220>
<223> Description of Artificial Sequence: position 77

<220>
<221> misc_feature
<222> (1)..(19)

<400> 229
tagtagactt cagagaact                                                    19


<210> 230
<211> 19
<212> DNA
<213> Artificial Sequence

<220>
<223> Description of Artificial Sequence: position 77

<220>
<221> misc_feature
<222> (1)..(19)

<400> 230
tagtagattt tagagaact                                                    19


<210> 231

<211> 19
<212> DNA
<213> Artificial Sequence

<220>
<223> Description of Artificial Sequence: position 77

<220>
<221> misc_feature
<222> (1)..(19)

<400> 231
tagtagattt cagagagct                                                    19


<210> 232
<211> 19
<212> DNA
<213> Artificial Sequence

<220>
<223> Description of Artificial Sequence: position 77

<220>
<221> misc_feature
<222> (1)..(19)

<400> 232
tagtggattt cagagaact                                                    19


<210> 233
<211> 19
<212> DNA
<213> Artificial Sequence

<220>
<223> Description of Artificial Sequence: position 77

<220>
<221> misc_feature
<222> (1)..(19)

<400> 233
tggtagattt cagagaact                                                    19


<210> 234
<211> 19
<212> DNA
<213> Artificial Sequence

<220>
<223> Description of Artificial Sequence: position 77

<220>
<221> misc_feature
<222> (1)..(19)

<400> 234
taatggattt cagagaact                                                    19


<210> 235
<211> 19
<212> DNA

<213> Artificial Sequence

<220>
<223> Description of Artificial Sequence: position 77

<220>
<221> misc_feature
<222> (1)..(19)

<400> 235
tagtagattt cagcgaact                                                    19


<210> 236
<211> 19
<212> DNA
<213> Artificial Sequence

<220>
<223> Description of Artificial Sequence: position 77

<220>
<221> misc_feature
<222> (1)..(19)

<400> 236
tggtagattt cagagagct                                                    19


<210> 237
<211> 19
<212> DNA
<213> Artificial Sequence

<220>
<223> Description of Artificial Sequence: position 77

<220>
<221> misc_feature
<222> (1)..(19)

<400> 237
tagtagattt tagggaact                                                    19


<210> 238
<211> 18
<212> DNA
<213> Artificial Sequence

<220>
<223> Description of Artificial Sequence: position 98

<220>
<221> misc_feature
<222> (1)..(18)

<400> 238
cacatcccgc agggttaa                                                     18


<210> 239
<211> 18
<212> DNA
<213> Artificial Sequence

<220>
<223> Description of Artificial Sequence: position 98

<220>
<221> misc_feature
<222> (1)..(18)

<400> 239
cacatcccgc agggttac                                                    18


<210> 240
<211> 18
<212> DNA
<213> Artificial Sequence

<220>
<223> Description of Artificial Sequence: position 98

<220>
<221> misc_feature
<222> (1)..(18)

<400> 240
cacatcccgc agggttga                                                    18


<210> 241
<211> 18
<212> DNA
<213> Artificial Sequence

<220>
<223> Description of Artificial Sequence: position 98

<220>
<221> misc_feature
<222> (1)..(18)

<400> 241
cacatcccgc agggtata                                                    18


<210> 242
<211> 18
<212> DNA
<213> Artificial Sequence

<220>
<223> Description of Artificial Sequence: position 98

<220>
<221> misc_feature
<222> (1)..(18)

<400> 242
cacatcccgc agggataa                                                    18


<210> 243
<211> 18
<212> DNA
<213> Artificial Sequence

<220>
<223> Description of Artificial Sequence: position 98

<220>
<221> misc_feature
<222> (1)..(18)

<400> 243
cacatcccgc agggctaa                                                    18


<210> 244
<211> 18
<212> DNA
<213> Artificial Sequence

<220>
<223> Description of Artificial Sequence: position 98

<220>
<221> misc_feature
<222> (1)..(18)

<400> 244
cacatcccgc agggctga                                                    18


<210> 245
<211> 18
<212> DNA
<213> Artificial Sequence

<220>
<223> Description of Artificial Sequence: position 98

<220>
<221> misc_feature
<222> (1)..(18)

<400> 245
cacatcccgc aggattaa                                                    18


<210> 246
<211> 18
<212> DNA
<213> Artificial Sequence

<220>
<223> Description of Artificial Sequence: position 98

<220>
<221> misc_feature
<222> (1)..(18)

<400> 246
cacatcccgc aggattac                                                    18


<210> 247
<211> 18
<212> DNA
<213> Artificial Sequence

<220>
<223> Description of Artificial Sequence: position 98

<220>

<221> misc_feature
<222> (1)..(18)

<400> 247
cacatcccgc aggactaa                                                    18


<210> 248
<211> 18
<212> DNA
<213> Artificial Sequence

<220>
<223> Description of Artificial Sequence: position 98

<220>
<221> misc_feature
<222> (1)..(18)

<400> 248
cacatcccgc aggtttaa                                                    18


<210> 249
<211> 18
<212> DNA
<213> Artificial Sequence

<220>
<223> Description of Artificial Sequence: position 98

<220>
<221> misc_feature
<222> (1)..(18)

<400> 249
cacatcccgc aggttaca                                                    18


<210> 250
<211> 18
<212> DNA
<213> Artificial Sequence

<220>
<223> Description of Artificial Sequence: position 98

<220>
<221> misc_feature
<222> (1)..(18)

<400> 250
cacatcccgc aggttgaa                                                    18


<210> 251
<211> 18
<212> DNA
<213> Artificial Sequence

<220>
<223> Description of Artificial Sequence: position 98

<220>
<221> misc_feature
<222> (1)..(18)

<400> 251
cacatcccgc agtttata                                                    18


<210> 252
<211> 18
<212> DNA
<213> Artificial Sequence

<220>
<223> Description of Artificial Sequence: position 98

<220>
<221> misc_feature
<222> (1)..(18)

<400> 252
cacatcccgc agttatat                                                    18


<210> 253
<211> 18
<212> DNA
<213> Artificial Sequence

<220>
<223> Description of Artificial Sequence: position 98

<220>
<221> misc_feature
<222> (1)..(18)

<400> 253
cacatcccgc ggggttaa                                                    18


<210> 254
<211> 18
<212> DNA
<213> Artificial Sequence

<220>
<223> Description of Artificial Sequence: position 98

<220>
<221> misc_feature
<222> (1)..(18)

<400> 254
cacatcccgc gggtttaa                                                    18


<210> 255
<211> 18
<212> DNA
<213> Artificial Sequence

<220>
<223> Description of Artificial Sequence: position 98

<220>
<221> misc_feature
<222> (1)..(18)

<400> 255

cacatcccgc tgggttaa                                                    18

<210> 256
<211> 18
<212> DNA
<213> Artificial Sequence

<220>
<223> Description of Artificial Sequence: position 98

<220>
<221> misc_feature
<222> (1)..(18)

<400> 256
cacatcccgc tgggctga                                                    18

<210> 257
<211> 18
<212> DNA
<213> Artificial Sequence

<220>
<223> Description of Artificial Sequence: position 98

<220>
<221> misc_feature
<222> (1)..(18)

<400> 257
cacatcccgg agggttaa                                                    18

<210> 258
<211> 18
<212> DNA
<213> Artificial Sequence

<220>
<223> Description of Artificial Sequence: position 98

<220>
<221> misc_feature
<222> (1)..(18)

<400> 258
cacatccctc agggttaa                                                    18

<210> 259
<211> 18
<212> DNA
<213> Artificial Sequence

<220>
<223> Description of Artificial Sequence: position 98

<220>
<221> misc_feature
<222> (1)..(18)

<400> 259
cacatccctc agggttga                                                    18

<210> 260
<211> 18
<212> DNA
<213> Artificial Sequence

<220>
<223> Description of Artificial Sequence: position 98

<220>
<221> misc_feature
<222> (1)..(18)

<400> 260
cacatccctc tgggttaa                                                    18


<210> 261
<211> 18
<212> DNA
<213> Artificial Sequence

<220>
<223> Description of Artificial Sequence: position 98

<220>
<221> misc_feature
<222> (1)..(18)

<400> 261
cacatccagc agggttaa                                                    18


<210> 262
<211> 18
<212> DNA
<213> Artificial Sequence

<220>
<223> Description of Artificial Sequence: position 98

<220>
<221> misc_feature
<222> (1)..(18)

<400> 262
cacatccagc agggctaa                                                    18


<210> 263
<211> 18
<212> DNA
<213> Artificial Sequence

<220>
<223> Description of Artificial Sequence: position 98

<220>
<221> misc_feature
<222> (1)..(18)

<400> 263
cacatccagc aggattga                                                    18


<210> 264

<211> 18
<212> DNA
<213> Artificial Sequence

<220>
<223> Description of Artificial Sequence: position 98

<220>
<221> misc_feature
<222> (1)..(18)

<400> 264
cacatccagc aggactaa                                                    18


<210> 265
<211> 18
<212> DNA
<213> Artificial Sequence

<220>
<223> Description of Artificial Sequence: position 100

<220>
<221> misc_feature
<222> (1)..(18)

<400> 265
ccgcagggtt aaaaaaga                                                    18


<210> 266
<211> 18
<212> DNA
<213> Artificial Sequence

<220>
<223> Description of Artificial Sequence: position 100

<220>
<221> misc_feature
<222> (1)..(18)

<400> 266
ccgcagggtt aaaaagga                                                    18


<210> 267
<211> 18
<212> DNA
<213> Artificial Sequence

<220>
<223> Description of Artificial Sequence: position 100

<220>
<221> misc_feature
<222> (1)..(18)

<400> 267
ccgcagggtt aaaacaga                                                    18


<210> 268
<211> 18
<212> DNA

<213> Artificial Sequence

<220>
<223> Description of Artificial Sequence: position 100

<220>
<221> misc_feature
<222> (1)..(18)

<400> 268
ccgcagggtt aaagaaga                                                    18


<210> 269
<211> 18
<212> DNA
<213> Artificial Sequence

<220>
<223> Description of Artificial Sequence: position 100

<220>
<221> misc_feature
<222> (1)..(18)

<400> 269
ccgcagggtt aaagaagg                                                    18


<210> 270
<211> 18
<212> DNA
<213> Artificial Sequence

<220>
<223> Description of Artificial Sequence: position 100

<220>
<221> misc_feature
<222> (1)..(18)

<400> 270
ccgcagggtt acaaaaga                                                    18


<210> 271
<211> 18
<212> DNA
<213> Artificial Sequence

<220>
<223> Description of Artificial Sequence: position 100

<220>
<221> misc_feature
<222> (1)..(18)

<400> 271
ccgcagggtt gaaaaaga                                                    18


<210> 272
<211> 18
<212> DNA
<213> Artificial Sequence

<220>
<223> Description of Artificial Sequence: position 100

<220>
<221> misc_feature
<222> (1)..(18)

<400> 272
ccgcagggta taaaaaga                                                    18


<210> 273
<211> 18
<212> DNA
<213> Artificial Sequence

<220>
<223> Description of Artificial Sequence: position 100

<220>
<221> misc_feature
<222> (1)..(18)

<400> 273
ccgcagggat aaaaaaga                                                    18


<210> 274
<211> 18
<212> DNA
<213> Artificial Sequence

<220>
<223> Description of Artificial Sequence: position 100

<220>
<221> misc_feature
<222> (1)..(18)

<400> 274
ccgcagggct aaaaaaga                                                    18


<210> 275
<211> 18
<212> DNA
<213> Artificial Sequence

<220>
<223> Description of Artificial Sequence: position 100

<220>
<221> misc_feature
<222> (1)..(18)

<400> 275
ccgcagggct aaaaagga                                                    18


<210> 276
<211> 18
<212> DNA
<213> Artificial Sequence

<220>
<223> Description of Artificial Sequence: position 100

<220>
<221> misc_feature
<222> (1)..(18)

<400> 276
ccgcagggct gaaaaaga                                                    18


<210> 277
<211> 18
<212> DNA
<213> Artificial Sequence

<220>
<223> Description of Artificial Sequence: position 100

<220>
<221> misc_feature
<222> (1)..(18)

<400> 277
ccgcaggatt aaaaaaga                                                    18


<210> 278
<211> 18
<212> DNA
<213> Artificial Sequence

<220>
<223> Description of Artificial Sequence: position 100

<220>
<221> misc_feature
<222> (1)..(18)

<400> 278
ccgcaggatt aaaacaga                                                    18


<210> 279
<211> 18
<212> DNA
<213> Artificial Sequence

<220>
<223> Description of Artificial Sequence: position 100

<220>
<221> misc_feature
<222> (1)..(18)

<400> 279
ccgcaggatt aaagaaga                                                    18


<210> 280
<211> 18
<212> DNA
<213> Artificial Sequence

<220>
<223> Description of Artificial Sequence: position 100

<220>

<221> misc_feature
<222> (1)..(18)

<400> 280
ccgcaggatt acaaaaga                                                    18


<210> 281
<211> 18
<212> DNA
<213> Artificial Sequence

<220>
<223> Description of Artificial Sequence: position 100

<220>
<221> misc_feature
<222> (1)..(18)

<400> 281
ccgcaggact aaaaaaga                                                    18


<210> 282
<211> 18
<212> DNA
<213> Artificial Sequence

<220>
<223> Description of Artificial Sequence: position 100

<220>
<221> misc_feature
<222> (1)..(18)

<400> 282
ccgcaggttt aaaaaaga                                                    18


<210> 283
<211> 18
<212> DNA
<213> Artificial Sequence

<220>
<223> Description of Artificial Sequence: position 100

<220>
<221> misc_feature
<222> (1)..(18)

<400> 283
ccgcaggtta caaaaaga                                                    18


<210> 284
<211> 18
<212> DNA
<213> Artificial Sequence

<220>
<223> Description of Artificial Sequence: position 100

<220>
<221> misc_feature
<222> (1)..(18)

<400> 284
ccgcaggttg aaaaaaga                                                    18


<210> 285
<211> 18
<212> DNA
<213> Artificial Sequence

<220>
<223> Description of Artificial Sequence: position 100

<220>
<221> misc_feature
<222> (1)..(18)

<400> 285
ccgcagttta taaaaaga                                                    18


<210> 286
<211> 18
<212> DNA
<213> Artificial Sequence

<220>
<223> Description of Artificial Sequence: position 100

<220>
<221> misc_feature
<222> (1)..(18)

<400> 286
ccgcagttat ataaaaga                                                    18


<210> 287
<211> 18
<212> DNA
<213> Artificial Sequence

<220>
<223> Description of Artificial Sequence: position 100

<220>
<221> misc_feature
<222> (1)..(18)

<400> 287
ccgcggggtt aaaaaaga                                                    18


<210> 288
<211> 18
<212> DNA
<213> Artificial Sequence

<220>
<223> Description of Artificial Sequence: position 100

<220>
<221> misc_feature
<222> (1)..(18)

<400> 288

ccgcggggtt aaaacaga                                                    18


<210> 289
<211> 19
<212> DNA
<213> Artificial Sequence

<220>
<223> Description of Artificial Sequence: position 103

<220>
<221> misc_feature
<222> (1)..(19)

<400> 289
taaaaaagaa aaaatcagt                                                   19


<210> 290
<211> 19
<212> DNA
<213> Artificial Sequence

<220>
<223> Description of Artificial Sequence: position 103

<220>
<221> misc_feature
<222> (1)..(19)

<400> 290
taaaaaagaa aaagtcagt                                                   19


<210> 291
<211> 19
<212> DNA
<213> Artificial Sequence

<220>
<223> Description of Artificial Sequence: position 103

<220>
<221> misc_feature
<222> (1)..(19)

<400> 291
taaagaagaa aaaatcagt                                                   19


<210> 292
<211> 19
<212> DNA
<213> Artificial Sequence

<220>
<223> Description of Artificial Sequence: position 103

<220>
<221> misc_feature
<222> (1)..(19)

<400> 292
taaaacagaa aaaatcagt                                                   19

<210> 293
<211> 19
<212> DNA
<213> Artificial Sequence

<220>
<223> Description of Artificial Sequence: position 103

<220>
<221> misc_feature
<222> (1)..(19)

<400> 293
taaaaaagaa gaaatcagt                                                    19


<210> 294
<211> 19
<212> DNA
<213> Artificial Sequence

<220>
<223> Description of Artificial Sequence: position 103

<220>
<221> misc_feature
<222> (1)..(19)

<400> 294
tgaaaaagaa aaaatcagt                                                    19


<210> 295
<211> 19
<212> DNA
<213> Artificial Sequence

<220>
<223> Description of Artificial Sequence: position 103

<220>
<221> misc_feature
<222> (1)..(19)

<400> 295
taaaaaggaa aaaatcagt                                                    19


<210> 296
<211> 19
<212> DNA
<213> Artificial Sequence

<220>
<223> Description of Artificial Sequence: position 103

<220>
<221> misc_feature
<222> (1)..(19)

<400> 296
taaaaaagag aaaatcagt                                                    19


<210> 297

<211> 19
<212> DNA
<213> Artificial Sequence

<220>
<223> Description of Artificial Sequence: position 103

<220>
<221> misc_feature
<222> (1)..(19)

<400> 297
tacaaaagaa aaaatcagt                                                    19


<210> 298
<211> 19
<212> DNA
<213> Artificial Sequence

<220>
<223> Description of Artificial Sequence: position 103

<220>
<221> misc_feature
<222> (1)..(19)

<400> 298
taaaaaagaa aagatcagt                                                    19


<210> 299
<211> 19
<212> DNA
<213> Artificial Sequence

<220>
<223> Description of Artificial Sequence: position 103

<220>
<221> misc_feature
<222> (1)..(19)

<400> 299
ataaaaagaa aaaatcagt                                                    19


<210> 300
<211> 19
<212> DNA
<213> Artificial Sequence

<220>
<223> Description of Artificial Sequence: position 103

<220>
<221> misc_feature
<222> (1)..(19)

<400> 300
taaaacagaa gaaatcagt                                                    19


<210> 301
<211> 19
<212> DNA

<213> Artificial Sequence

<220>
<223> Description of Artificial Sequence: position 103

<220>
<221> misc_feature
<222> (1)..(19)

<400> 301
taaaaaagaa gaagtcagt                                                    19


<210> 302
<211> 19
<212> DNA
<213> Artificial Sequence

<220>
<223> Description of Artificial Sequence: position 103

<220>
<221> misc_feature
<222> (1)..(19)

<400> 302
tgaaaaagaa aaagtcagt                                                    19


<210> 303
<211> 19
<212> DNA
<213> Artificial Sequence

<220>
<223> Description of Artificial Sequence: position 103

<220>
<221> misc_feature
<222> (1)..(19)

<400> 303
taaaaaagaa aaattcagt                                                    19


<210> 304
<211> 19
<212> DNA
<213> Artificial Sequence

<220>
<223> Description of Artificial Sequence: position 106

<220>
<221> misc_feature
<222> (1)..(19)

<400> 304
aaaaatcagt aacagtact                                                    19


<210> 305
<211> 19
<212> DNA
<213> Artificial Sequence

<220>
<223> Description of Artificial Sequence: position 106

<220>
<221> misc_feature
<222> (1)..(19)

<400> 305
aaaagtcagt aacagtact                                                    19


<210> 306
<211> 19
<212> DNA
<213> Artificial Sequence

<220>
<223> Description of Artificial Sequence: position 106

<220>
<221> misc_feature
<222> (1)..(19)

<400> 306
agaaatcagt aacagtact                                                    19


<210> 307
<211> 19
<212> DNA
<213> Artificial Sequence

<220>
<223> Description of Artificial Sequence: position 106

<220>
<221> misc_feature
<222> (1)..(19)

<400> 307
aaaaatcagt aacagtatt                                                    19


<210> 308
<211> 19
<212> DNA
<213> Artificial Sequence

<220>
<223> Description of Artificial Sequence: position 106

<220>
<221> misc_feature
<222> (1)..(19)

<400> 308
aaaaatcagt aacagtgct                                                    19


<210> 309
<211> 19
<212> DNA
<213> Artificial Sequence

<220>
<223> Description of Artificial Sequence: position 106

<220>
<221> misc_feature
<222> (1)..(19)

<400> 309
aaaaatcagt gacagtact                                                    19


<210> 310
<211> 19
<212> DNA
<213> Artificial Sequence

<220>
<223> Description of Artificial Sequence: position 106

<220>
<221> misc_feature
<222> (1)..(19)

<400> 310
aaaaatcagt aacagtgtt                                                    19


<210> 311
<211> 19
<212> DNA
<213> Artificial Sequence

<220>
<223> Description of Artificial Sequence: position 106

<220>
<221> misc_feature
<222> (1)..(19)

<400> 311
aaagatcagt aacagtact                                                    19


<210> 312
<211> 19
<212> DNA
<213> Artificial Sequence

<220>
<223> Description of Artificial Sequence: position 106

<220>
<221> misc_feature
<222> (1)..(19)

<400> 312
gaaaatcagt aacagtact                                                    19


<210> 313
<211> 19
<212> DNA
<213> Artificial Sequence

<220>
<223> Description of Artificial Sequence: position 106

<220>

<221> misc_feature
<222> (1)..(19)

<400> 313
aaaaatcagt aacagtcct                                                    19


<210> 314
<211> 19
<212> DNA
<213> Artificial Sequence

<220>
<223> Description of Artificial Sequence: position 106

<220>
<221> misc_feature
<222> (1)..(19)

<400> 314
aaaaatcagt cacagtact                                                    19


<210> 315
<211> 19
<212> DNA
<213> Artificial Sequence

<220>
<223> Description of Artificial Sequence: position 106

<220>
<221> misc_feature
<222> (1)..(19)

<400> 315
aaaattcagt aacagtact                                                    19


<210> 316
<211> 19
<212> DNA
<213> Artificial Sequence

<220>
<223> Description of Artificial Sequence: position 106

<220>
<221> misc_feature
<222> (1)..(19)

<400> 316
acaaatcagt aacagtact                                                    19


<210> 317
<211> 19
<212> DNA
<213> Artificial Sequence

<220>
<223> Description of Artificial Sequence: position 106

<220>
<221> misc_feature
<222> (1)..(19)

<400> 317
aaaaatcagt aaccgtcct                                                    19


<210> 318
<211> 19
<212> DNA
<213> Artificial Sequence

<220>
<223> Description of Artificial Sequence: position 106

<220>
<221> misc_feature
<222> (1)..(19)

<400> 318
aaaaatcagt aacagtctt                                                    19


<210> 319
<211> 19
<212> DNA
<213> Artificial Sequence

<220>
<223> Description of Artificial Sequence: position 106

<220>
<221> misc_feature
<222> (1)..(19)

<400> 319
aaaagtcagt aacagtgct                                                    19


<210> 320
<211> 19
<212> DNA
<213> Artificial Sequence

<220>
<223> Description of Artificial Sequence: position 106

<220>
<221> misc_feature
<222> (1)..(19)

<400> 320
aaaaatcagt gacagtatt                                                    19


<210> 321
<211> 19
<212> DNA
<213> Artificial Sequence

<220>
<223> Description of Artificial Sequence: position 106

<220>
<221> misc_feature
<222> (1)..(19)

<400> 321

aaaaatcagt aacagttct                                                    19

<210> 322
<211> 19
<212> DNA
<213> Artificial Sequence

<220>
<223> Description of Artificial Sequence: position 108

<220>
<221> misc_feature
<222> (1)..(19)

<400> 322
cagtaacagt actggatgt                                                    19

<210> 323
<211> 19
<212> DNA
<213> Artificial Sequence

<220>
<223> Description of Artificial Sequence: position 108

<220>
<221> misc_feature
<222> (1)..(19)

<400> 323
cagtaacagt actagatgt                                                    19

<210> 324
<211> 19
<212> DNA
<213> Artificial Sequence

<220>
<223> Description of Artificial Sequence: position 108

<220>
<221> misc_feature
<222> (1)..(19)

<400> 324
cagtaacagt attggatgt                                                    19

<210> 325
<211> 19
<212> DNA
<213> Artificial Sequence

<220>
<223> Description of Artificial Sequence: position 108

<220>
<221> misc_feature
<222> (1)..(19)

<400> 325
cagtaacagt gctggatgt                                                    19

<210> 326
<211> 19
<212> DNA
<213> Artificial Sequence

<220>
<223> Description of Artificial Sequence: position 108

<220>
<221> misc_feature
<222> (1)..(19)

<400> 326
cagtgacagt actggatgt                                                    19


<210> 327
<211> 19
<212> DNA
<213> Artificial Sequence

<220>
<223> Description of Artificial Sequence: position 108

<220>
<221> misc_feature
<222> (1)..(19)

<400> 327
cagtaacagt cctggatgt                                                    19


<210> 328
<211> 19
<212> DNA
<213> Artificial Sequence

<220>
<223> Description of Artificial Sequence: position 108

<220>
<221> misc_feature
<222> (1)..(19)

<400> 328
cagtaacagt gctagatgt                                                    19


<210> 329
<211> 19
<212> DNA
<213> Artificial Sequence

<220>
<223> Description of Artificial Sequence: position 108

<220>
<221> misc_feature
<222> (1)..(19)

<400> 329
cagtaacagt gttggatgt                                                    19


<210> 330

<211> 19
<212> DNA
<213> Artificial Sequence

<220>
<223> Description of Artificial Sequence: position 108

<220>
<221> misc_feature
<222> (1)..(19)

<400> 330
cagtaacagt gttagatgt                                                    19


<210> 331
<211> 19
<212> DNA
<213> Artificial Sequence

<220>
<223> Description of Artificial Sequence: position 108

<220>
<221> misc_feature
<222> (1)..(19)

<400> 331
cagtaacagt actggacgt                                                    19


<210> 332
<211> 19
<212> DNA
<213> Artificial Sequence

<220>
<223> Description of Artificial Sequence: position 108

<220>
<221> misc_feature
<222> (1)..(19)

<400> 332
cagtcacagt actagatgt                                                    19


<210> 333
<211> 19
<212> DNA
<213> Artificial Sequence

<220>
<223> Description of Artificial Sequence: position 108

<220>
<221> misc_feature
<222> (1)..(19)

<400> 333
cagtaaccgt cctggatgt                                                    19


<210> 334
<211> 19
<212> DNA

<213> Artificial Sequence

<220>
<223> Description of Artificial Sequence: position 108

<220>
<221> misc_feature
<222> (1)..(19)

<400> 334
cagtaacagt cttggatgt                                                    19


<210> 335
<211> 19
<212> DNA
<213> Artificial Sequence

<220>
<223> Description of Artificial Sequence: position 108

<220>
<221> misc_feature
<222> (1)..(19)

<400> 335
cagtgacagt cctggatgt                                                    19


<210> 336
<211> 19
<212> DNA
<213> Artificial Sequence

<220>
<223> Description of Artificial Sequence: position 108

<220>
<221> misc_feature
<222> (1)..(19)

<400> 336
cagtgacagt attggatgt                                                    19


<210> 337
<211> 19
<212> DNA
<213> Artificial Sequence

<220>
<223> Description of Artificial Sequence: position 108

<220>
<221> misc_feature
<222> (1)..(19)

<400> 337
cagtaacagt tctggatgt                                                    19


<210> 338
<211> 19
<212> DNA
<213> Artificial Sequence

<220>
<223> Description of Artificial Sequence: position 115

<220>
<221> misc_feature
<222> (1)..(19)

<400> 338
gtgatgcata tttttcagt                                                    19


<210> 339
<211> 19
<212> DNA
<213> Artificial Sequence

<220>
<223> Description of Artificial Sequence: position 115

<220>
<221> misc_feature
<222> (1)..(19)

<400> 339
gtgatgcata tttttcaat                                                    19


<210> 340
<211> 19
<212> DNA
<213> Artificial Sequence

<220>
<223> Description of Artificial Sequence: position 115

<220>
<221> misc_feature
<222> (1)..(19)

<400> 340
gggatgcata tttttcagt                                                    19


<210> 341
<211> 19
<212> DNA
<213> Artificial Sequence

<220>
<223> Description of Artificial Sequence: position 115

<220>
<221> misc_feature
<222> (1)..(19)

<400> 341
gtgatgcata tttctcagt                                                    19


<210> 342
<211> 19
<212> DNA
<213> Artificial Sequence

<220>
<223> Description of Artificial Sequence: position 115

<220>
<221> misc_feature
<222> (1)..(19)

<400> 342
gtgatgcata cttttcagt                                                    19


<210> 343
<211> 19
<212> DNA
<213> Artificial Sequence

<220>
<223> Description of Artificial Sequence: position 115

<220>
<221> misc_feature
<222> (1)..(19)

<400> 343
gagatgcata tttttcagt                                                    19


<210> 344
<211> 19
<212> DNA
<213> Artificial Sequence

<220>
<223> Description of Artificial Sequence: position 115

<220>
<221> misc_feature
<222> (1)..(19)

<400> 344
gcgatgcata tttttcagt                                                    19


<210> 345
<211> 19
<212> DNA
<213> Artificial Sequence

<220>
<223> Description of Artificial Sequence: position 115

<220>
<221> misc_feature
<222> (1)..(19)

<400> 345
gtgatgcata tttctcaat                                                    19


<210> 346
<211> 19
<212> DNA
<213> Artificial Sequence

<220>
<223> Description of Artificial Sequence: position 115

<220>

<221> misc_feature
<222> (1)..(19)

<400> 346
gagatgcata tttctcagt                                                    19


<210> 347
<211> 19
<212> DNA
<213> Artificial Sequence

<220>
<223> Description of Artificial Sequence: position 115

<220>
<221> misc_feature
<222> (1)..(19)

<400> 347
gagatgcata cttttcagt                                                    19


<210> 348
<211> 19
<212> DNA
<213> Artificial Sequence

<220>
<223> Description of Artificial Sequence: position 115

<220>
<221> misc_feature
<222> (1)..(19)

<400> 348
gtgatgcata cttttcaat                                                    19


<210> 349
<211> 19
<212> DNA
<213> Artificial Sequence

<220>
<223> Description of Artificial Sequence: position 115

<220>
<221> misc_feature
<222> (1)..(19)

<400> 349
gagatgcata tttttcaat                                                    19


<210> 350
<211> 19
<212> DNA
<213> Artificial Sequence

<220>
<223> Description of Artificial Sequence: position 115

<220>
<221> misc_feature
<222> (1)..(19)

<400> 350
gtgacgcata tttttcagt                                                    19


<210> 351
<211> 19
<212> DNA
<213> Artificial Sequence

<220>
<223> Description of Artificial Sequence: position 115

<220>
<221> misc_feature
<222> (1)..(19)

<400> 351
gtgatgcata tttttccat                                                    19


<210> 352
<211> 19
<212> DNA
<213> Artificial Sequence

<220>
<223> Description of Artificial Sequence: position 115

<220>
<221> misc_feature
<222> (1)..(19)

<400> 352
gggatgcata tttctcagt                                                    19


<210> 353
<211> 19
<212> DNA
<213> Artificial Sequence

<220>
<223> Description of Artificial Sequence: position 116

<220>
<221> misc_feature
<222> (1)..(19)

<400> 353
atgcatattt ttcagttcc                                                    19


<210> 354
<211> 19
<212> DNA
<213> Artificial Sequence

<220>
<223> Description of Artificial Sequence: position 116

<220>
<221> misc_feature
<222> (1)..(19)

<400> 354

atgcatattt ttcaattcc                                                    19

<210> 355
<211> 19
<212> DNA
<213> Artificial Sequence

<220>
<223> Description of Artificial Sequence: position 116

<220>
<221> misc_difference
<222> (1)..(19)

<400> 355
atgcatattt ctcagttcc                                                    19

<210> 356
<211> 19
<212> DNA
<213> Artificial Sequence

<220>
<223> Description of Artificial Sequence: position 116

<220>
<221> misc_feature
<222> (1)..(19)

<400> 356
atgcatactt ttcagttcc                                                    19

<210> 357
<211> 19
<212> DNA
<213> Artificial Sequence

<220>
<223> Description of Artificial Sequence: position 116

<220>
<221> misc_feature
<222> (1)..(19)

<400> 357
atgcatattt ttcagtccc                                                    19

<210> 358
<211> 19
<212> DNA
<213> Artificial Sequence

<220>
<223> Description of Artificial Sequence: position 116

<220>
<221> misc_feature
<222> (1)..(19)

<400> 358
atgcatattt ttcagtgcc                                                    19

<210> 359
<211> 19
<212> DNA
<213> Artificial Sequence

<220>
<223> Description of Artificial Sequence: position 116

<220>
<221> misc_feature
<222> (1)..(19)

<400> 359
atgcatattt ctcaattcc                                                    19


<210> 360
<211> 19
<212> DNA
<213> Artificial Sequence

<220>
<223> Description of Artificial Sequence: position 116

<220>
<221> misc_feature
<222> (1)..(19)

<400> 360
acgcatattt ttcagttcc                                                    19


<210> 361
<211> 19
<212> DNA
<213> Artificial Sequence

<220>
<223> Description of Artificial Sequence: position 116

<220>
<221> misc_feature
<222> (1)..(19)

<400> 361
atgcatactt ttcaattcc                                                    19


<210> 362
<211> 19
<212> DNA
<213> Artificial Sequence

<220>
<223> Description of Artificial Sequence: position 116

<220>
<221> misc_feature
<222> (1)..(19)

<400> 362
atgcatattt ttcagtacc                                                    19


<210> 363

<211> 19
<212> DNA
<213> Artificial Sequence

<220>
<223> Description of Artificial Sequence: position 116

<220>
<221> misc_feature
<222> (1)..(19)

<400> 363
atgcatattt ttccattcc                                                    19


<210> 364
<211> 19
<212> DNA
<213> Artificial Sequence

<220>
<223> Description of Artificial Sequence: position 151

<220>
<221> misc_feature
<222> (1)..(19)

<400> 364
tgcttccaca gggatggaa                                                    19


<210> 365
<211> 19
<212> DNA
<213> Artificial Sequence

<220>
<223> Description of Artificial Sequence: position 151

<220>
<221> misc_feature
<222> (1)..(19)

<400> 365
tgctgccaca gggatggaa                                                    19


<210> 366
<211> 19
<212> DNA
<213> Artificial Sequence

<220>
<223> Description of Artificial Sequence: position 151

<220>
<221> misc_feature
<222> (1)..(19)

<400> 366
tgcttccaca aggatggaa                                                    19


<210> 367
<211> 19
<212> DNA

<213> Artificial Sequence

<220>
<223> Description of Artificial Sequence: position 151

<220>
<221> misc_feature
<222> (1)..(19)

<400> 367
tgctcccaca gggatggaa                                                    19


<210> 368
<211> 19
<212> DNA
<213> Artificial Sequence

<220>
<223> Description of Artificial Sequence: position 151

<220>
<221> misc_feature
<222> (1)..(19)

<400> 368
tacttccaca gggatggaa                                                    19


<210> 369
<211> 19
<212> DNA
<213> Artificial Sequence

<220>
<223> Description of Artificial Sequence: position 151

<220>
<221> misc_feature
<222> (1)..(19)

<400> 369
tgcttccgca gggatggaa                                                    19


<210> 370
<211> 19
<212> DNA
<213> Artificial Sequence

<220>
<223> Description of Artificial Sequence: position 151

<220>
<221> misc_feature
<222> (1)..(19)

<400> 370
tgctaccaca gggatggaa                                                    19


<210> 371
<211> 19
<212> DNA
<213> Artificial Sequence

<220>
<223> Description of Artificial Sequence: position 151

<220>
<221> misc_feature
<222> (1)..(19)

<400> 371
tgcttccaca ggggtggaa                                                    19


<210> 372
<211> 19
<212> DNA
<213> Artificial Sequence

<220>
<223> Description of Artificial Sequence: position 151

<220>
<221> misc_feature
<222> (1)..(19)

<400> 372
tgcttccgca aggatggaa                                                    19


<210> 373
<211> 19
<212> DNA
<213> Artificial Sequence

<220>
<223> Description of Artificial Sequence: position 151

<220>
<221> misc_feature
<222> (1)..(19)

<400> 373
tgctgccaca aggatggaa                                                    19


<210> 374
<211> 19
<212> DNA
<213> Artificial Sequence

<220>
<223> Description of Artificial Sequence: position 151

<220>
<221> misc_feature
<222> (1)..(19)

<400> 374
tacttccgca gggatggaa                                                    19


<210> 375
<211> 19
<212> DNA
<213> Artificial Sequence

<220>
<223> Description of Artificial Sequence: position 151

<220>
<221> misc_feature
<222> (1)..(19)

<400> 375
tgcttcccca gggatggaa                                                    19


<210> 376
<211> 19
<212> DNA
<213> Artificial Sequence

<220>
<223> Description of Artificial Sequence: position 151

<220>
<221> misc_feature
<222> (1)..(19)

<400> 376
ctgttccgca ggggtggaa                                                    19


<210> 377
<211> 19
<212> DNA
<213> Artificial Sequence

<220>
<223> Description of Artificial Sequence: position 151

<220>
<221> misc_feature
<222> (1)..(19)

<400> 377
tgcgtccaca gggatggaa                                                    19


<210> 378
<211> 18
<212> DNA
<213> Artificial Sequence

<220>
<223> Description of Artificial Sequence: position 181

<220>
<221> misc_feature
<222> (1)..(18)

<400> 378
tagttatcta tcaataca                                                     18


<210> 379
<211> 18
<212> DNA
<213> Artificial Sequence

<220>
<223> Description of Artificial Sequence: position 181

<220>

<221> misc_feature
<222> (1)..(18)

<400> 379
tagttatcta tcaatacg                                                    18


<210> 380
<211> 18
<212> DNA
<213> Artificial Sequence

<220>
<223> Description of Artificial Sequence: position 181

<220>
<221> misc_feature
<222> (1)..(18)

<400> 380
tggttatcta tcaataca                                                    18


<210> 381
<211> 18
<212> DNA
<213> Artificial Sequence

<220>
<223> Description of Artificial Sequence: position 181

<220>
<221> misc_feature
<222> (1)..(18)

<400> 381
tagttattta tcaataca                                                    18


<210> 382
<211> 18
<212> DNA
<213> Artificial Sequence

<220>
<223> Description of Artificial Sequence: position 181

<220>
<221> misc_feature
<222> (1)..(18)

<400> 382
tagttatcta tcaatata                                                    18


<210> 383
<211> 18
<212> DNA
<213> Artificial Sequence

<220>
<223> Description of Artificial Sequence: position 181

<220>
<221> misc_feature
<222> (1)..(18)

<400> 383
tagtcatcta tcaataca                                                    18


<210> 384
<211> 18
<212> DNA
<213> Artificial Sequence

<220>
<223> Description of Artificial Sequence: position 181

<220>
<221> misc_feature
<222> (1)..(18)

<400> 384
tagttatcta tcagtaca                                                    18


<210> 385
<211> 18
<212> DNA
<213> Artificial Sequence

<220>
<223> Description of Artificial Sequence: position 181

<220>
<221> misc_feature
<222> (1)..(18)

<400> 385
tggttatcta tcaataca                                                    18


<210> 386
<211> 18
<212> DNA
<213> Artificial Sequence

<220>
<223> Description of Artificial Sequence: position 181

<220>
<221> misc_feature
<222> (1)..(18)

<400> 386
tggtgatcta ccaatata                                                    18


<210> 387
<211> 18
<212> DNA
<213> Artificial Sequence

<220>
<223> Description of Artificial Sequence: position 181

<220>
<221> misc_feature
<222> (1)..(18)

<400> 387

tagttatcta ccaataca                                                    18

<210> 388
<211> 18
<212> DNA
<213> Artificial Sequence

<220>
<223> Description of Artificial Sequence: position 181

<220>
<221> misc_feature
<222> (1)..(18)

<400> 388
tagttattta tcaatacg                                                    18

<210> 389
<211> 18
<212> DNA
<213> Artificial Sequence

<220>
<223> Description of Artificial Sequence: position 181

<220>
<221> misc_feature
<222> (1)..(18)

<400> 389
tagtcatcta tcaatacg                                                    18

<210> 390
<211> 18
<212> DNA
<213> Artificial Sequence

<220>
<223> Description of Artificial Sequence: position 181

<220>
<221> misc_feature
<222> (1)..(18)

<400> 390
tagttatctg tcaatacg                                                    18

<210> 391
<211> 18
<212> DNA
<213> Artificial Sequence

<220>
<223> Description of Artificial Sequence: position 181

<220>
<221> misc_feature
<222> (1)..(18)

<400> 391
tagtgatcta ccaataca                                                    18

<210> 392
<211> 18
<212> DNA
<213> Artificial Sequence

<220>
<223> Description of Artificial Sequence: position 181

<220>
<221> misc_feature
<222> (1)..(18)

<400> 392
tagtgatcta ccaatata                                                    18


<210> 393
<211> 18
<212> DNA
<213> Artificial Sequence

<220>
<223> Description of Artificial Sequence: position 181

<220>
<221> misc_feature
<222> (1)..(18)

<400> 393
tagttatcta ccagtaca                                                    18


<210> 394
<211> 18
<212> DNA
<213> Artificial Sequence

<220>
<223> Description of Artificial Sequence: position 181

<220>
<221> misc_feature
<222> (1)..(18)

<400> 394
tagttatctg tcaataca                                                    18


<210> 395
<211> 18
<212> DNA
<213> Artificial Sequence

<220>
<223> Description of Artificial Sequence: position 181

<220>
<221> misc_feature
<222> (1)..(18)

<400> 395
tagtgatcta tcaatacg                                                    18


<210> 396

<211> 18
<212> DNA
<213> Artificial Sequence

<220>
<223> Description of Artificial Sequence: position 181

<220>
<221> misc_feature
<222> (1)..(18)

<400> 396
tagttatcta tcagtacg                                                    18


<210> 397
<211> 18
<212> DNA
<213> Artificial Sequence

<220>
<223> Description of Artificial Sequence: position 181

<220>
<221> misc_feature
<222> (1)..(18)

<400> 397
tagttatcta ccaatacg                                                    18


<210> 398
<211> 18
<212> DNA
<213> Artificial Sequence

<220>
<223> Description of Artificial Sequence: position 181

<220>
<221> misc_feature
<222> (1)..(18)

<400> 398
tagtgatcta tcaatata                                                    18


<210> 399
<211> 18
<212> DNA
<213> Artificial Sequence

<220>
<223> Description of Artificial Sequence: position 181

<220>
<221> misc_feature
<222> (1)..(18)

<400> 399
tagatatcta tcaatata                                                    18


<210> 400
<211> 18
<212> DNA

<213> Artificial Sequence

<220>
<223> Description of Artificial Sequence: position 181

<220>
<221> misc_feature
<222> (1)..(18)

<400> 400
tggtgatcta tcaataca                                                    18


<210> 401
<211> 18
<212> DNA
<213> Artificial Sequence

<220>
<223> Description of Artificial Sequence: position 181

<220>
<221> misc_feature
<222> (1)..(18)

<400> 401
tggtgatcta ccaataca                                                    18


<210> 402
<211> 19
<212> DNA
<213> Artificial Sequence

<220>
<223> Description of Artificial Sequence: position 184

<220>
<221> misc_feature
<222> (1)..(19)

<400> 402
atcaatacat ggatgattt                                                   19


<210> 403
<211> 19
<212> DNA
<213> Artificial Sequence

<220>
<223> Description of Artificial Sequence: position 184

<220>
<221> misc_feature
<222> (1)..(19)

<400> 403
atcaatacgt ggatgattt                                                   19


<210> 404
<211> 19
<212> DNA
<213> Artificial Sequence

<220>
<223> Description of Artificial Sequence: position 184

<220>
<221> misc_feature
<222> (1)..(19)

<400> 404
atcaatacat ggatgactt                                                    19


<210> 405
<211> 19
<212> DNA
<213> Artificial Sequence

<220>
<223> Description of Artificial Sequence: position 184

<220>
<221> misc_feature
<222> (1)..(19)

<400> 405
atcaatatat ggatgattt                                                    19


<210> 406
<211> 19
<212> DNA
<213> Artificial Sequence

<220>
<223> Description of Artificial Sequence: position 184

<220>
<221> misc_feature
<222> (1)..(19)

<400> 406
accaatacat ggatgattt                                                    19


<210> 407
<211> 19
<212> DNA
<213> Artificial Sequence

<220>
<223> Description of Artificial Sequence: position 184

<220>
<221> misc_feature
<222> (1)..(19)

<400> 407
accaatatat ggatgattt                                                    19


<210> 408
<211> 19
<212> DNA
<213> Artificial Sequence

<220>
<223> Description of Artificial Sequence: position 184

<220>
<221> misc_feature
<222> (1)..(19)

<400> 408
atcagtacat ggatgattt                                                    19


<210> 409
<211> 19
<212> DNA
<213> Artificial Sequence

<220>
<223> Description of Artificial Sequence: position 184

<220>
<221> misc_feature
<222> (1)..(19)

<400> 409
atcaatacat ggatgatct                                                    19


<210> 410
<211> 19
<212> DNA
<213> Artificial Sequence

<220>
<223> Description of Artificial Sequence: position 184

<220>
<221> misc_feature
<222> (1)..(19)

<400> 410
atcaatacgt ggatgatct                                                    19


<210> 411
<211> 19
<212> DNA
<213> Artificial Sequence

<220>
<223> Description of Artificial Sequence: position 184

<220>
<221> misc_feature
<222> (1)..(19)

<400> 411
atcaatacgt ggatgactt                                                    19


<210> 412
<211> 19
<212> DNA
<213> Artificial Sequence

<220>
<223> Description of Artificial Sequence: position 184

<220>

<221> misc_feature
<222> (1)..(19)

<400> 412
atcagtacgt ggatgattt                                                    19


<210> 413
<211> 19
<212> DNA
<213> Artificial Sequence

<220>
<223> Description of Artificial Sequence: position 184

<220>
<221> misc_feature
<222> (1)..(19)

<400> 413
atcaatacat agatgattt                                                    19


<210> 414
<211> 19
<212> DNA
<213> Artificial Sequence

<220>
<223> Description of Artificial Sequence: position 184

<220>
<221> misc_feature
<222> (1)..(19)

<400> 414
accaatacgt ggatgattt                                                    19


<210> 415
<211> 19
<212> DNA
<213> Artificial Sequence

<220>
<223> Description of Artificial Sequence: position 184

<220>
<221> misc_feature
<222> (1)..(19)

<400> 415
accagtacat ggatgattt                                                    19


<210> 416
<211> 19
<212> DNA
<213> Artificial Sequence

<220>
<223> Description of Artificial Sequence: position 184

<220>
<221> misc_feature
<222> (1)..(19)

<400> 416
atcaatacat ggatgacct                                                    19


<210> 417
<211> 19
<212> DNA
<213> Artificial Sequence

<220>
<223> Description of Artificial Sequence: position 184

<220>
<221> misc_feature
<222> (1)..(19)

<400> 417
gtcaatacat ggatgattt                                                    19


<210> 418
<211> 19
<212> DNA
<213> Artificial Sequence

<220>
<223> Description of Artificial Sequence: position 184

<220>
<221> misc_feature
<222> (1)..(19)

<400> 418
gtcaatacgt ggatgatct                                                    19


<210> 419
<211> 19
<212> DNA
<213> Artificial Sequence

<220>
<223> Description of Artificial Sequence: position 184

<220>
<221> misc_feature
<222> (1)..(19)

<400> 419
atcaatacat agatgatct                                                    19


<210> 420
<211> 19
<212> DNA
<213> Artificial Sequence

<220>
<223> Description of Artificial Sequence: position 184

<220>
<221> misc_feature
<222> (1)..(19)

<400> 420

atcaatacat ggacgactt                                                    19

<210> 421
<211> 19
<212> DNA
<213> Artificial Sequence

<220>
<223> Description of Artificial Sequence: position 184

<220>
<221> misc_feature
<222> (1)..(19)

<400> 421
atcaatatgt ggatgattt                                                    19

<210> 422
<211> 19
<212> DNA
<213> Artificial Sequence

<220>
<223> Description of Artificial Sequence: position 188

<220>
<221> misc_feature
<222> (1)..(19)

<400> 422
atgatttgta tgtaggatc                                                    19

<210> 423
<211> 19
<212> DNA
<213> Artificial Sequence

<220>
<223> Description of Artificial Sequence: position 188

<220>
<221> misc_feature
<222> (1)..(19)

<400> 423
atgatttata tgtaggatc                                                    19

<210> 424
<211> 19
<212> DNA
<213> Artificial Sequence

<220>
<223> Description of Artificial Sequence: position 188

<220>
<221> misc_feature
<222> (1)..(19)

<400> 424
atgacttgta tgtaggatc                                                    19

<210> 425
<211> 19
<212> DNA
<213> Artificial Sequence

<220>
<223> Description of Artificial Sequence: position 188

<220>
<221> misc_feature
<222> (1)..(19)

<400> 425
atgatctgta tgtaggatc                                                    19


<210> 426
<211> 19
<212> DNA
<213> Artificial Sequence

<220>
<223> Description of Artificial Sequence: position 188

<220>
<221> misc_feature
<222> (1)..(19)

<400> 426
atgatttgta tgtagggtc                                                    19


<210> 427
<211> 19
<212> DNA
<213> Artificial Sequence

<220>
<223> Description of Artificial Sequence: position 188

<220>
<221> misc_feature
<222> (1)..(19)

<400> 427
atgatttgta tataggatc                                                    19


<210> 428
<211> 19
<212> DNA
<213> Artificial Sequence

<220>
<223> Description of Artificial Sequence: position 188

<220>
<221> misc_feature
<222> (1)..(19)

<400> 428
atgatttgta tgtaggctc                                                    19


<210> 429

<211> 19
<212> DNA
<213> Artificial Sequence

<220>
<223> Description of Artificial Sequence: position 188

<220>
<221> misc_feature
<222> (1)..(19)

<400> 429
atgatctgta cgtaggatc                                                    19


<210> 430
<211> 19
<212> DNA
<213> Artificial Sequence

<220>
<223> Description of Artificial Sequence: position 188

<220>
<221> misc_feature
<222> (1)..(19)

<400> 430
atgatttata tataggatc                                                    19


<210> 431
<211> 19
<212> DNA
<213> Artificial Sequence

<220>
<223> Description of Artificial Sequence: position 188

<220>
<221> misc_feature
<222> (1)..(19)

<400> 431
atgatttgta cgtaggatc                                                    19


<210> 432
<211> 19
<212> DNA
<213> Artificial Sequence

<220>
<223> Description of Artificial Sequence: position 188

<220>
<221> misc_feature
<222> (1)..(19)

<400> 432
atgacttata tgtaggatc                                                    19


<210> 433
<211> 19
<212> DNA

<213> Artificial Sequence

<220>
<223> Description of Artificial Sequence: position 188

<220>
<221> misc_feature
<222> (1)..(19)

<400> 433
atgacctgta tgtaggatc                                                    19


<210> 434
<211> 19
<212> DNA
<213> Artificial Sequence

<220>
<223> Description of Artificial Sequence: position 188

<220>
<221> misc_feature
<222> (1)..(19)

<400> 434
atgatttgta tgtgggatc                                                    19


<210> 435
<211> 19
<212> DNA
<213> Artificial Sequence

<220>
<223> Description of Artificial Sequence: position 188

<220>
<221> misc_feature
<222> (1)..(19)

<400> 435
atgatttgta tgtagcatc                                                    19


<210> 436
<211> 19
<212> DNA
<213> Artificial Sequence

<220>
<223> Description of Artificial Sequence: position 188

<220>
<221> misc_feature
<222> (1)..(19)

<400> 436
acgacttgta tgtaggatc                                                    19


<210> 437
<211> 19
<212> DNA
<213> Artificial Sequence

<220>
<223> Description of Artificial Sequence: position 188

<220>
<221> misc_feature
<222> (1)..(19)

<400> 437
atgatttgtt ggtaggatc                                                    19


<210> 438
<211> 19
<212> DNA
<213> Artificial Sequence

<220>
<223> Description of Artificial Sequence: position 190

<220>
<221> misc_feature
<222> (1)..(19)

<400> 438
tgtatgtagg atctgactt                                                    19


<210> 439
<211> 19
<212> DNA
<213> Artificial Sequence

<220>
<223> Description of Artificial Sequence: position 190

<220>
<221> misc_feature
<222> (1)..(19)

<400> 439
tgtatgtagg atctgattt                                                    19


<210> 440
<211> 19
<212> DNA
<213> Artificial Sequence

<220>
<223> Description of Artificial Sequence: position 190

<220>
<221> misc_feature
<222> (1)..(19)

<400> 440
tatatgtagg atctgactt                                                    19


<210> 441
<211> 19
<212> DNA
<213> Artificial Sequence

<220>
<223> Description of Artificial Sequence: position 190

<220>
<221> misc_feature
<222> (1)..(19)

<400> 441
tgtatgtagg gtctgactt                                                    19


<210> 442
<211> 19
<212> DNA
<213> Artificial Sequence

<220>
<223> Description of Artificial Sequence: position 190

<220>
<221> misc_feature
<222> (1)..(19)

<400> 442
tatatgtagg atctgattt                                                    19


<210> 443
<211> 19
<212> DNA
<213> Artificial Sequence

<220>
<223> Description of Artificial Sequence: position 190

<220>
<221> misc_feature
<222> (1)..(19)

<400> 443
tgtatgtagg atctgacct                                                    19


<210> 444
<211> 19
<212> DNA
<213> Artificial Sequence

<220>
<223> Description of Artificial Sequence: position 190

<220>
<221> misc_feature
<222> (1)..(19)

<400> 444
tgtatgtagg ctctgactt                                                    19


<210> 445
<211> 19
<212> DNA
<213> Artificial Sequence

<220>
<223> Description of Artificial Sequence: position 190

<220>

<221> misc_feature
<222> (1)..(19)

<400> 445
tgtatgtagg atcagactt                                                    19


<210> 446
<211> 19
<212> DNA
<213> Artificial Sequence

<220>
<223> Description of Artificial Sequence: position 190

<220>
<221> misc_feature
<222> (1)..(19)

<400> 446
tatatgtagg atcagactt                                                    19


<210> 447
<211> 19
<212> DNA
<213> Artificial Sequence

<220>
<223> Description of Artificial Sequence: position 190

<220>
<221> misc_feature
<222> (1)..(19)

<400> 447
tgtacgtagg atctgattt                                                    19


<210> 448
<211> 19
<212> DNA
<213> Artificial Sequence

<220>
<223> Description of Artificial Sequence: position 190

<220>
<221> misc_feature
<222> (1)..(19)

<400> 448
tgtatatagg atctgactt                                                    19


<210> 449
<211> 19
<212> DNA
<213> Artificial Sequence

<220>
<223> Description of Artificial Sequence: position 190

<220>
<221> misc_feature
<222> (1)..(19)

<400> 449
tatatatagg atctgactt                                                    19


<210> 450
<211> 19
<212> DNA
<213> Artificial Sequence

<220>
<223> Description of Artificial Sequence: position 190

<220>
<221> misc_feature
<222> (1)..(19)

<400> 450
tgtatgtagg atcggactt                                                    19


<210> 451
<211> 19
<212> DNA
<213> Artificial Sequence

<220>
<223> Description of Artificial Sequence: position 190

<220>
<221> misc_feature
<222> (1)..(19)

<400> 451
tgtatgtggg atctgactt                                                    19


<210> 452
<211> 19
<212> DNA
<213> Artificial Sequence

<220>
<223> Description of Artificial Sequence: position 190

<220>
<221> misc_feature
<222> (1)..(19)

<400> 452
tgtatatagg atcagactt                                                    19


<210> 453
<211> 19
<212> DNA
<213> Artificial Sequence

<220>
<223> Description of Artificial Sequence: position 190

<220>
<221> misc_feature
<222> (1)..(19)

<400> 453

tgtacgtagg atctgactt                                                    19

<210> 454
<211> 19
<212> DNA
<213> Artificial Sequence

<220>
<223> Description of Artificial Sequence: position 190

<220>
<221> misc_feature
<222> (1)..(19)

<400> 454
tgtatgtagg gtctgattt                                                    19

<210> 455
<211> 18
<212> DNA
<213> Artificial Sequence

<220>
<223> Description of Artificial Sequence: position 210

<220>
<221> misc_feature
<222> (1)..(18)

<400> 455
aacatctgtt gaggtggg                                                     18

<210> 456
<211> 18
<212> DNA
<213> Artificial Sequence

<220>
<223> Description of Artificial Sequence: position 210

<220>
<221> misc_feature
<222> (1)..(18)

<400> 456
aacatctgtt gaagtggg                                                     18

<210> 457
<211> 18
<212> DNA
<213> Artificial Sequence

<220>
<223> Description of Artificial Sequence: position 210

<220>
<221> misc_feature
<222> (1)..(18)

<400> 457
aacatctgtg gaggtggg                                                     18

<210> 458
<211> 18
<212> DNA
<213> Artificial Sequence

<220>
<223> Description of Artificial Sequence: position 210

<220>
<221> misc_feature
<222> (1)..(18)

<400> 458
aatatctgtg gaggtggg                                                    18


<210> 459
<211> 18
<212> DNA
<213> Artificial Sequence

<220>
<223> Description of Artificial Sequence: position 210

<220>
<221> misc_feature
<222> (1)..(18)

<400> 459
aacatttgtt gaggtggg                                                    18


<210> 460
<211> 18
<212> DNA
<213> Artificial Sequence

<220>
<223> Description of Artificial Sequence: position 210

<220>
<221> misc_feature
<222> (1)..(18)

<400> 460
agcatctgtt gaggtggg                                                    18


<210> 461
<211> 18
<212> DNA
<213> Artificial Sequence

<220>
<223> Description of Artificial Sequence: position 210

<220>
<221> misc_feature
<222> (1)..(18)

<400> 461
aacatctact gagatggg                                                    18


<210> 462

<211> 18
<212> DNA
<213> Artificial Sequence

<220>
<223> Description of Artificial Sequence: position 210

<220>
<221> misc_feature
<222> (1)..(18)

<400> 462
aacatttgtt gaagtggg                                                    18


<210> 463
<211> 18
<212> DNA
<213> Artificial Sequence

<220>
<223> Description of Artificial Sequence: position 210

<220>
<221> misc_feature
<222> (1)..(18)

<400> 463
aacatctgtt gagatggg                                                    18


<210> 464
<211> 18
<212> DNA
<213> Artificial Sequence

<220>
<223> Description of Artificial Sequence: position 210

<220>
<221> misc_feature
<222> (1)..(18)

<400> 464
aacatttgtt ggggtggg                                                    18


<210> 465
<211> 18
<212> DNA
<213> Artificial Sequence

<220>
<223> Description of Artificial Sequence: position 210

<220>
<221> misc_feature
<222> (1)..(18)

<400> 465
agcatctgtt gaagtggg                                                    18


<210> 466
<211> 18
<212> DNA

<213> Artificial Sequence

<220>
<223> Description of Artificial Sequence: position 210

<220>
<221> misc_feature
<222> (1)..(18)

<400> 466
aacatctgtt ggggtggg                                                    18


<210> 467
<211> 18
<212> DNA
<213> Artificial Sequence

<220>
<223> Description of Artificial Sequence: position 210

<220>
<221> misc_feature
<222> (1)..(18)

<400> 467
gacatctgtt gaggtggg                                                    18


<210> 468
<211> 18
<212> DNA
<213> Artificial Sequence

<220>
<223> Description of Artificial Sequence: position 210

<220>
<221> misc_feature
<222> (1)..(18)

<400> 468
aacatctgtt gaaatggg                                                    18


<210> 469
<211> 18
<212> DNA
<213> Artificial Sequence

<220>
<223> Description of Artificial Sequence: position 210

<220>
<221> misc_feature
<222> (1)..(18)

<400> 469
aacatctgtg gaagtggg                                                    18


<210> 470
<211> 18
<212> DNA
<213> Artificial Sequence

<220>
<223> Description of Artificial Sequence: position 210

<220>
<221> misc_feature
<222> (1)..(18)

<400> 470
accatctgtt gaagtggg                                                    18


<210> 471
<211> 18
<212> DNA
<213> Artificial Sequence

<220>
<223> Description of Artificial Sequence: position 210

<220>
<221> misc_feature
<222> (1)..(18)

<400> 471
ctcatctatt gagctggg                                                    18


<210> 472
<211> 18
<212> DNA
<213> Artificial Sequence

<220>
<223> Description of Artificial Sequence: position 210

<220>
<221> misc_feature
<222> (1)..(18)

<400> 472
aacatctgtt aaagtggg                                                    18


<210> 473
<211> 18
<212> DNA
<213> Artificial Sequence

<220>
<223> Description of Artificial Sequence: position 210

<220>
<221> misc_feature
<222> (1)..(18)

<400> 473
aacatttgtt aaagtggg                                                    18


<210> 474
<211> 18
<212> DNA
<213> Artificial Sequence

<220>
<223> Description of Artificial Sequence: position 210

<220>
<221> misc_feature
<222> (1)..(18)

<400> 474
aacacttgtt gaggtggg                                                    18


<210> 475
<211> 18
<212> DNA
<213> Artificial Sequence

<220>
<223> Description of Artificial Sequence: position 210

<220>
<221> misc_feature
<222> (1)..(18)

<400> 475
aatatttgtg gaggtggg                                                    18


<210> 476
<211> 18
<212> DNA
<213> Artificial Sequence

<220>
<223> Description of Artificial Sequence: position 210

<220>
<221> misc_feature
<222> (1)..(18)

<400> 476
aatatttgtg gagatggg                                                    18


<210> 477
<211> 18
<212> DNA
<213> Artificial Sequence

<220>
<223> Description of Artificial Sequence: position 210

<220>
<221> misc_feature
<222> (1)..(18)

<400> 477
aacatctgtt ggcgtggg                                                    18


<210> 478
<211> 18
<212> DNA
<213> Artificial Sequence

<220>
<223> Description of Artificial Sequence: position 210

<220>

<221> misc_feature
<222> (1)..(18)

<400> 478
aacatttgtg gaggtggg                                                    18


<210> 479
<211> 18
<212> DNA
<213> Artificial Sequence

<220>
<223> Description of Artificial Sequence: position 210

<220>
<221> misc_feature
<222> (1)..(18)

<400> 479
aatatctatg gaggtggg                                                    18


<210> 480
<211> 18
<212> DNA
<213> Artificial Sequence

<220>
<223> Description of Artificial Sequence: position 210

<220>
<221> misc_feature
<222> (1)..(18)

<400> 480
accatctgtt gaggtggg                                                    18


<210> 481
<211> 18
<212> DNA
<213> Artificial Sequence

<220>
<223> Description of Artificial Sequence: position 210

<220>
<221> misc_feature
<222> (1)..(18)

<400> 481
agcatctgtt ggggtggg                                                    18


<210> 482
<211> 18
<212> DNA
<213> Artificial Sequence

<220>
<223> Description of Artificial Sequence: position 210

<220>
<221> misc_feature
<222> (1)..(18)

<400> 482
agcatctgtt aaagtggg                                                    18


<210> 483
<211> 18
<212> DNA
<213> Artificial Sequence

<220>
<223> Description of Artificial Sequence: position 210

<220>
<221> misc_feature
<222> (1)..(18)

<400> 483
agcatctgtg gaggtggg                                                    18


<210> 484
<211> 18
<212> DNA
<213> Artificial Sequence

<220>
<223> Description of Artificial Sequence: position 210

<220>
<221> misc_feature
<222> (1)..(18)

<400> 484
agcacctgtt gaagtggg                                                    18


<210> 485
<211> 18
<212> DNA
<213> Artificial Sequence

<220>
<223> Description of Artificial Sequence: position 210

<220>
<221> misc_feature
<222> (1)..(18)

<400> 485
agtatctgtg gaggtggg                                                    18


<210> 486
<211> 18
<212> DNA
<213> Artificial Sequence

<220>
<223> Description of Artificial Sequence: position 210

<220>
<221> misc_feature
<222> (1)..(18)

<400> 486

atcatctgtt gaagtggg                                                    18

<210> 487
<211> 18
<212> DNA
<213> Artificial Sequence

<220>
<223> Description of Artificial Sequence: position 210

<220>
<221> misc_feature
<222> (1)..(18)

<400> 487
aacatctgtt gagttggg                                                    18

<210> 488
<211> 18
<212> DNA
<213> Artificial Sequence

<220>
<223> Description of Artificial Sequence: position 210

<220>
<221> misc_feature
<222> (1)..(18)

<400> 488
aacatctgtt gacgtggg                                                    18

<210> 489
<211> 18
<212> DNA
<213> Artificial Sequence

<220>
<223> Description of Artificial Sequence: position 210

<220>
<221> misc_feature
<222> (1)..(18)

<400> 489
aacatctgtt acagtggg                                                    18

<210> 490
<211> 18
<212> DNA
<213> Artificial Sequence

<220>
<223> Description of Artificial Sequence: position 210

<220>
<221> misc_feature
<222> (1)..(18)

<400> 490
aacatctatt gaggtggg                                                    18

<210> 491
<211> 18
<212> DNA
<213> Artificial Sequence

<220>
<223> Description of Artificial Sequence: position 210

<220>
<221> misc_feature
<222> (1)..(18)

<400> 491
aacatctatt gaagtggg                                                    18


<210> 492
<211> 18
<212> DNA
<213> Artificial Sequence

<220>
<223> Description of Artificial Sequence: position 210

<220>
<221> misc_feature
<222> (1)..(18)

<400> 492
aacacctgtg gaggtggg                                                    18


<210> 493
<211> 18
<212> DNA
<213> Artificial Sequence

<220>
<223> Description of Artificial Sequence: position 210

<220>
<221> misc_feature
<222> (1)..(18)

<400> 493
aatatctgtt gaggtggg                                                    18


<210> 494
<211> 18
<212> DNA
<213> Artificial Sequence

<220>
<223> Description of Artificial Sequence: position 210

<220>
<221> misc_feature
<222> (1)..(18)

<400> 494
agcatctgtt aaggtggg                                                    18


<210> 495

<211> 18
<212> DNA
<213> Artificial Sequence

<220>
<223> Description of Artificial Sequence: position 210

<220>
<221> misc_feature
<222> (1)..(18)

<400> 495
agcatttgtt gaagtggg                                                    18


<210> 496
<211> 18
<212> DNA
<213> Artificial Sequence

<220>
<223> Description of Artificial Sequence: position 210

<220>
<221> misc_feature
<222> (1)..(18)

<400> 496
gacatctgtt ggggtggg                                                    18


<210> 497
<211> 18
<212> DNA
<213> Artificial Sequence

<220>
<223> Description of Artificial Sequence: position 210

<220>
<221> misc_feature
<222> (1)..(18)

<400> 497
gatatctgtg gaggtggg                                                    18


<210> 498
<211> 18
<212> DNA
<213> Artificial Sequence

<220>
<223> Description of Artificial Sequence: position 215

<220>
<221> misc_feature
<222> (1)..(18)

<400> 498
tggggattta ccacacca                                                    18


<210> 499
<211> 18
<212> DNA

<213> Artificial Sequence

<220>
<223> Description of Artificial Sequence: position 215

<220>
<221> misc_feature
<222> (1)..(18)

<400> 499
tggggatttt ccacacca                                                    18


<210> 500
<211> 18
<212> DNA
<213> Artificial Sequence

<220>
<223> Description of Artificial Sequence: position 215

<220>
<221> misc_feature
<222> (1)..(18)

<400> 500
tgggggttta ccacacca                                                    18


<210> 501
<211> 18
<212> DNA
<213> Artificial Sequence

<220>
<223> Description of Artificial Sequence: position 215

<220>
<221> misc_feature
<222> (1)..(18)

<400> 501
tggggactta ccacacca                                                    18


<210> 502
<211> 18
<212> DNA
<213> Artificial Sequence

<220>
<223> Description of Artificial Sequence: position 215

<220>
<221> misc_feature
<222> (1)..(18)

<400> 502
tgggggtttt ccacacca                                                    18


<210> 503
<211> 18
<212> DNA
<213> Artificial Sequence

<220>
<223> Description of Artificial Sequence: position 215

<220>
<221> misc_feature
<222> (1)..(18)

<400> 503
tggggatttt tcacacca                                                    18


<210> 504
<211> 18
<212> DNA
<213> Artificial Sequence

<220>
<223> Description of Artificial Sequence: position 215

<220>
<221> misc_feature
<222> (1)..(18)

<400> 504
tggggattta ccacccca                                                    18


<210> 505
<211> 18
<212> DNA
<213> Artificial Sequence

<220>
<223> Description of Artificial Sequence: position 215

<220>
<221> misc_feature
<222> (1)..(18)

<400> 505
tggggatttt acacacca                                                    18


<210> 506
<211> 18
<212> DNA
<213> Artificial Sequence

<220>
<223> Description of Artificial Sequence: position 215

<220>
<221> misc_feature
<222> (1)..(18)

<400> 506
tgggggctta ccacacca                                                    18


<210> 507
<211> 18
<212> DNA
<213> Artificial Sequence

<220>
<223> Description of Artificial Sequence: position 215

<220>
<221> misc_feature
<222> (1)..(18)

<400> 507
tggggattca ccacacca                                                    18


<210> 508
<211> 18
<212> DNA
<213> Artificial Sequence

<220>
<223> Description of Artificial Sequence: position 215

<220>
<221> misc_feature
<222> (1)..(18)

<400> 508
tggggattaa ccacacca                                                    18


<210> 509
<211> 18
<212> DNA
<213> Artificial Sequence

<220>
<223> Description of Artificial Sequence: position 215

<220>
<221> misc_feature
<222> (1)..(18)

<400> 509
tggggattct tcacacca                                                    18


<210> 510
<211> 18
<212> DNA
<213> Artificial Sequence

<220>
<223> Description of Artificial Sequence: position 215

<220>
<221> misc_feature
<222> (1)..(18)

<400> 510
tgggggttta ctacacca                                                    18


<210> 511
<211> 18
<212> DNA
<213> Artificial Sequence

<220>
<223> Description of Artificial Sequence: position 215

<220>

<221> misc_feature
<222> (1)..(18)

<400> 511
tgggggtttt acacacca                                                    18


<210> 512
<211> 18
<212> DNA
<213> Artificial Sequence

<220>
<223> Description of Artificial Sequence: position 215

<220>
<221> misc_feature
<222> (1)..(18)

<400> 512
tggggattta ccacgcca                                                    18


<210> 513
<211> 18
<212> DNA
<213> Artificial Sequence

<220>
<223> Description of Artificial Sequence: position 215

<220>
<221> misc_feature
<222> (1)..(18)

<400> 513
tgggggttca ccacacca                                                    18


<210> 514
<211> 18
<212> DNA
<213> Artificial Sequence

<220>
<223> Description of Artificial Sequence: position 215

<220>
<221> misc_feature
<222> (1)..(18)

<400> 514
tggggattta ccacaccc                                                    18


<210> 515
<211> 18
<212> DNA
<213> Artificial Sequence

<220>
<223> Description of Artificial Sequence: position 215

<220>
<221> misc_feature
<222> (1)..(18)

<400> 515
tggggattta ctacacca                                                    18


<210> 516
<211> 18
<212> DNA
<213> Artificial Sequence

<220>
<223> Description of Artificial Sequence: position 215

<220>
<221> misc_feature
<222> (1)..(18)

<400> 516
   tggggattta ctacccca                                                   18


<210> 517
<211> 18
<212> DNA
<213> Artificial Sequence

<220>
<223> Description of Artificial Sequence: position 215

<220>
<221> misc_feature
<222> (1)..(18)

<400> 517
tggggattta acacacca                                                    18


<210> 518
<211> 18
<212> DNA
<213> Artificial Sequence

<220>
<223> Description of Artificial Sequence: position 215

<220>
<221> misc_feature
<222> (1)..(18)

<400> 518
tggggatttt ctacacca                                                    18


<210> 519
<211> 18
<212> DNA
<213> Artificial Sequence

<220>
<223> Description of Artificial Sequence: position 215

<220>
<221> misc_feature
<222> (1)..(18)

<400> 519

tggggacttt tcacacca                                                    18

<210> 520
<211> 18
<212> DNA
<213> Artificial Sequence

<220>
<223> Description of Artificial Sequence: position 215

<220>
<221> misc_feature
<222> (1)..(18)

<400> 520
tggggactca ccacacca                                                    18

<210> 521
<211> 18
<212> DNA
<213> Artificial Sequence

<220>
<223> Description of Artificial Sequence: position 215

<220>
<221> misc_difference
<222> (1)..(18)

<400> 521
tgggggttga ccacacca                                                    18

<210> 522
<211> 18
<212> DNA
<213> Artificial Sequence

<220>
<223> Description of Artificial Sequence: position 219

<220>
<221> misc_feature
<222> (1)..(18)

<400> 522
caccagacaa aaaacatc                                                    18

<210> 523
<211> 18
<212> DNA
<213> Artificial Sequence

<220>
<223> Description of Artificial Sequence: position 219

<220>
<221> misc_feature
<222> (1)..(18)

<400> 523
caccagataa aaaacatc                                                    18

<210> 524
<211> 18
<212> DNA
<213> Artificial Sequence

<220>
<223> Description of Artificial Sequence: position 219

<220>
<221> misc_feature
<222> (1)..(18)

<400> 524
caccagacaa aaagcatc
18

<210> 525
<211> 18
<212> DNA
<213> Artificial Sequence

<220>
<223> Description of Artificial Sequence: position 219

<220>
<221> misc_feature
<222> (1)..(18)

<400> 525
caccagacca aaaacatc
18

<210> 526
<211> 18
<212> DNA
<213> Artificial Sequence

<220>
<223> Description of Artificial Sequence: position 219

<220>
<221> misc_feature
<222> (1)..(18)

<400> 526
caccagacaa aaaacacc
18

<210> 527
<211> 18
<212> DNA
<213> Artificial Sequence

<220>
<223> Description of Artificial Sequence: position 219

<220>
<221> misc_feature
<222> (1)..(18)

<400> 527
caccagaaca aaaacatc
18

<210> 528

<211> 18
<212> DNA
<213> Artificial Sequence

<220>
<223> Description of Artificial Sequence: position 219

<220>
<221> misc_feature
<222> (1)..(18)

<400> 528
ccccagacaa aaaacatc                                                    18


<210> 529
<211> 18
<212> DNA
<213> Artificial Sequence

<220>
<223> Description of Artificial Sequence: position 219

<220>
<221> misc_feature
<222> (1)..(18)

<400> 529
caccagacaa gaaacatc                                                    18


<210> 530
<211> 18
<212> DNA
<213> Artificial Sequence

<220>
<223> Description of Artificial Sequence: position 219

<220>
<221> misc_feature
<222> (1)..(18)

<400> 530
caccagacaa caaacatc                                                    18


<210> 531
<211> 18
<212> DNA
<213> Artificial Sequence

<220>
<223> Description of Artificial Sequence: position 219

<220>
<221> misc_feature
<222> (1)..(18)

<400> 531
caccagacga aaaacatc                                                    18


<210> 532
<211> 18
<212> DNA

<213> Artificial Sequence

<220>
<223> Description of Artificial Sequence: position 219

<220>
<221> misc_feature
<222> (1)..(18)

<400> 532
caccagacaa caagcatc                                                    18


<210> 533
<211> 18
<212> DNA
<213> Artificial Sequence

<220>
<223> Description of Artificial Sequence: position 219

<220>
<221> misc_feature
<222> (1)..(18)

<400> 533
caccagacaa gaaacacc                                                    18


<210> 534
<211> 18
<212> DNA
<213> Artificial Sequence

<220>
<223> Description of Artificial Sequence: position 219

<220>
<221> misc_feature
<222> (1)..(18)

<400> 534
caccagacga gaaacatc                                                    18


<210> 535
<211> 18
<212> DNA
<213> Artificial Sequence

<220>
<223> Description of Artificial Sequence: position 219

<220>
<221> misc_feature
<222> (1)..(18)

<400> 535
caccagataa aaagcatc                                                    18


<210> 536
<211> 18
<212> DNA
<213> Artificial Sequence

<220>
<223> Description of Artificial Sequence: position 219

<220>
<221> misc_feature
<222> (1)..(18)

<400> 536
cgccagacaa aaaacatc                                                    18


<210> 537
<211> 18
<212> DNA
<213> Artificial Sequence

<220>
<223> Description of Artificial Sequence: position 219

<220>
<221> misc_feature
<222> (1)..(18)

<400> 537
caccagacaa gaagcatc                                                    18


<210> 538
<211> 18
<212> DNA
<213> Artificial Sequence

<220>
<223> Description of Artificial Sequence: position 219

<220>
<221> misc_feature
<222> (1)..(18)

<400> 538
caccagacag aaaacatc                                                    18


<210> 539
<211> 18
<212> DNA
<213> Artificial Sequence

<220>
<223> Description of Artificial Sequence: position 219

<220>
<221> misc_feature
<222> (1)..(18)

<400> 539
caccagaaca aaaacacc                                                    18


<210> 540
<211> 18
<212> DNA
<213> Artificial Sequence

<220>
<223> Description of Artificial Sequence: position 219

<220>
<221> misc_feature
<222> (1)..(18)

<400> 540
caccagataa gaaacatc                                                    18


<210> 541
<211> 18
<212> DNA
<213> Artificial Sequence

<220>
<223> Description of Artificial Sequence: position 219

<220>
<221> misc_feature
<222> (1)..(18)

<400> 541
cacccgacaa aaaacatc                                                    18


<210> 542
<211> 18
<212> DNA
<213> Artificial Sequence

<220>
<223> Description of Artificial Sequence: position 219

<220>
<221> misc_feature
<222> (1)..(18)

<400> 542
caccggacaa aaagcatc                                                    18


<210> 543
<211> 18
<212> DNA
<213> Artificial Sequence

<220>
<223> Description of Artificial Sequence: position 219

<220>
<221> misc_feature
<222> (1)..(18)

<400> 543
ccccagacaa aaagcatc                                                    18

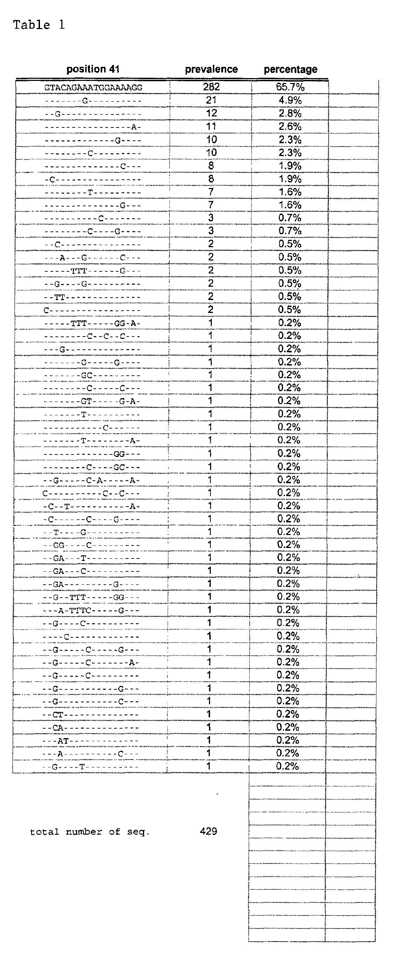

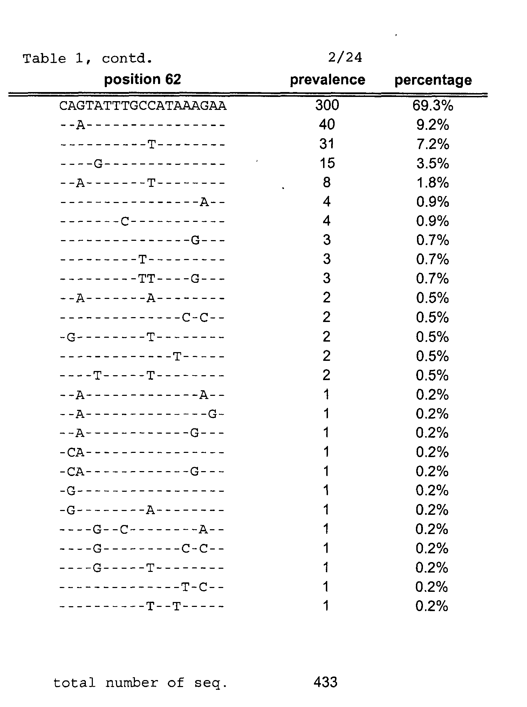

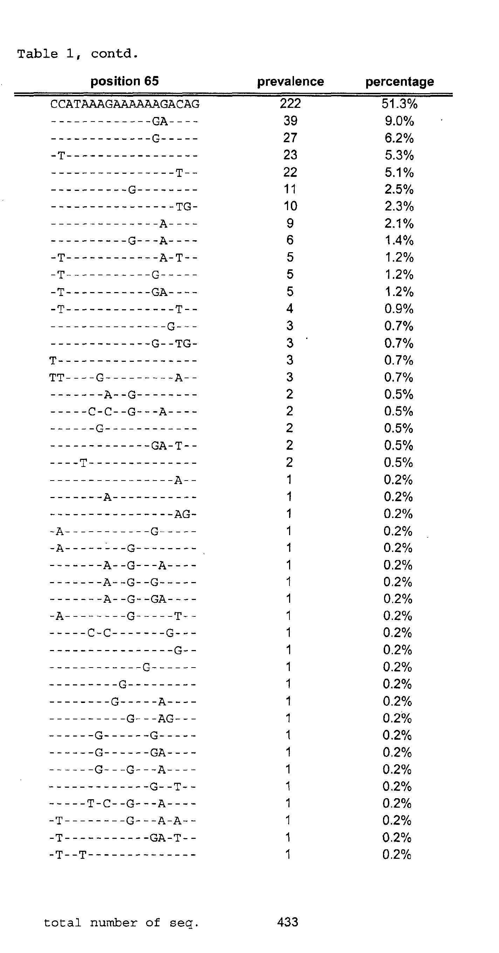

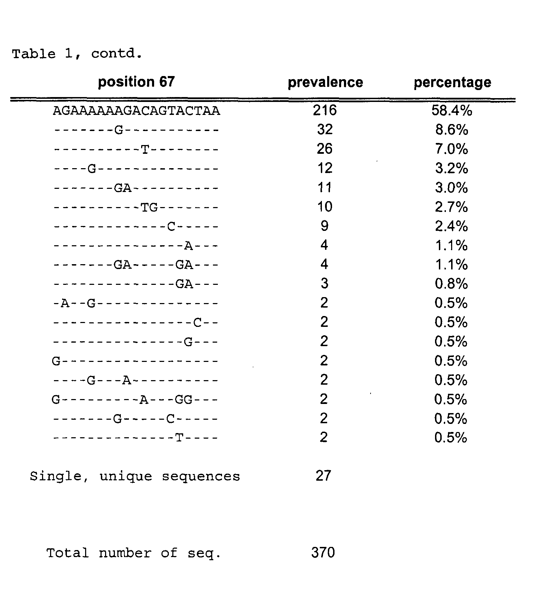

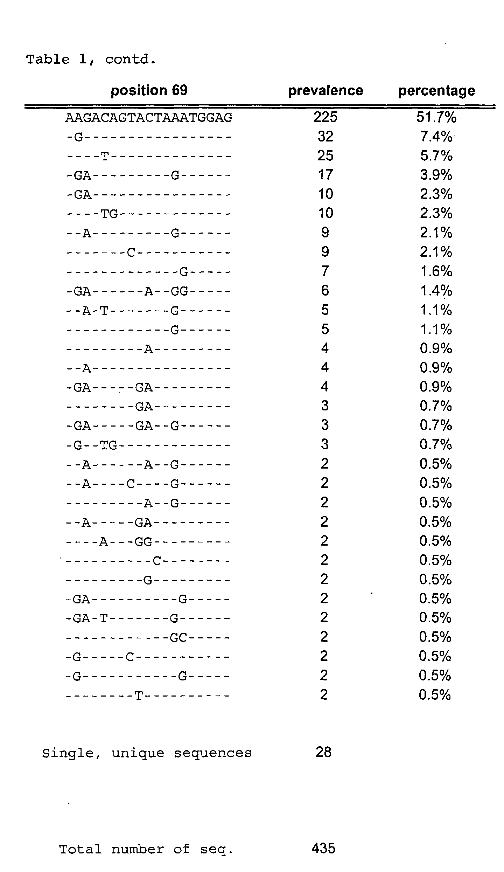

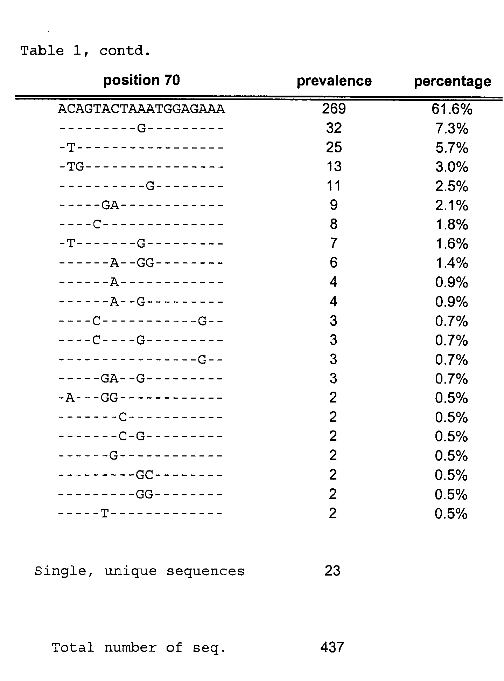

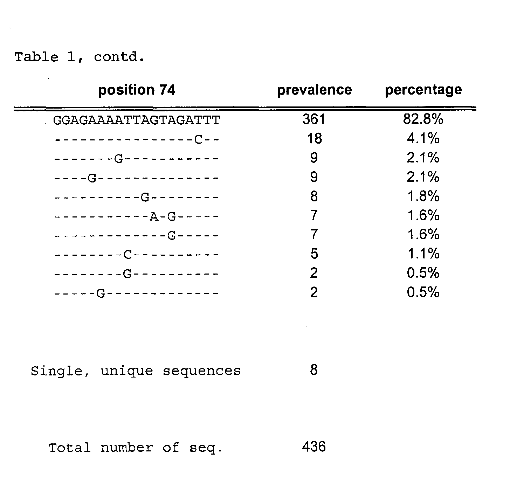

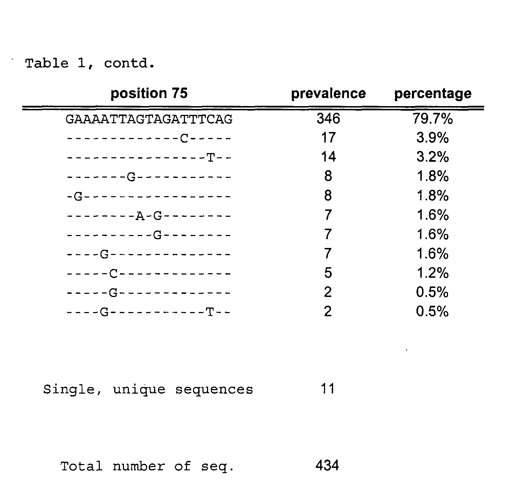

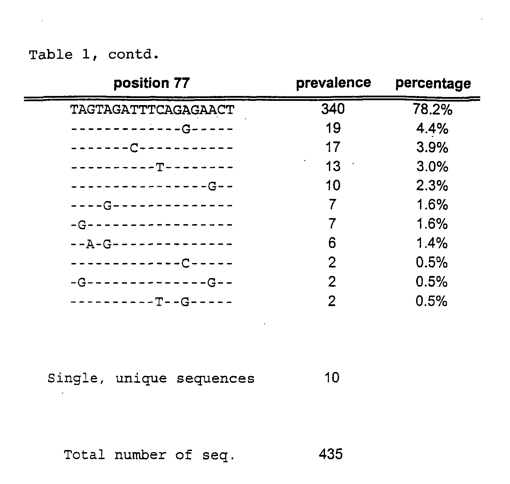

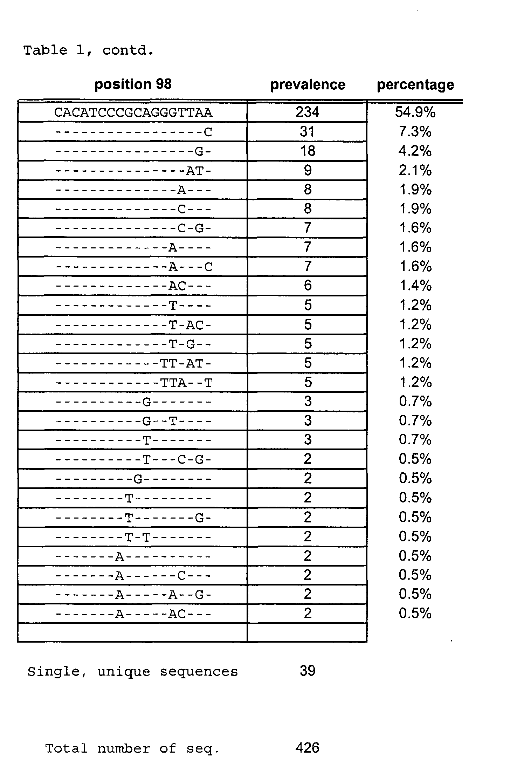

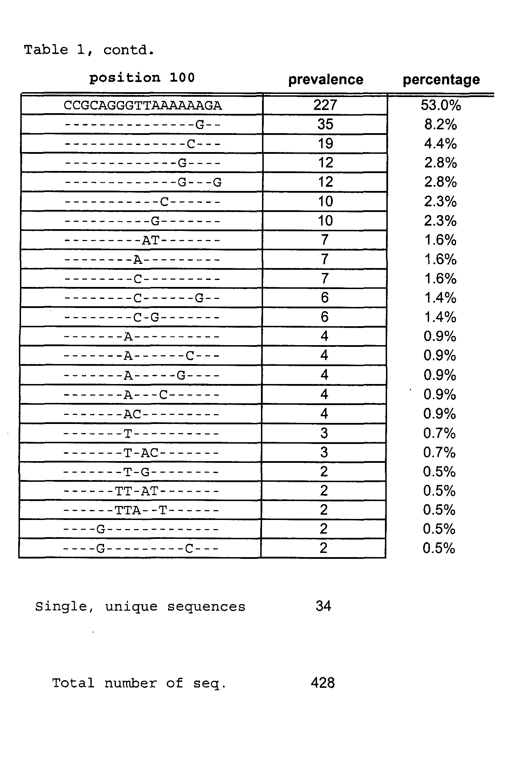

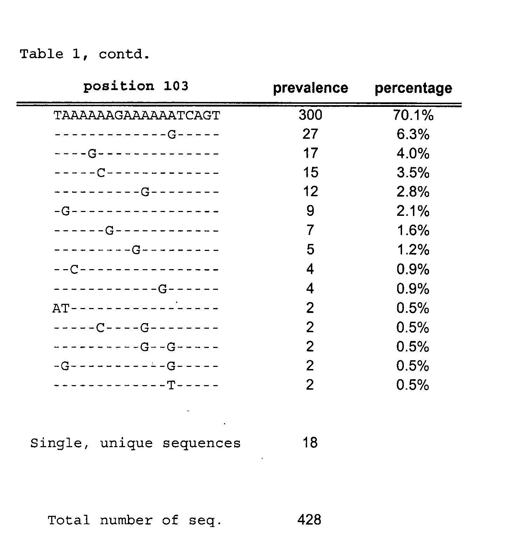

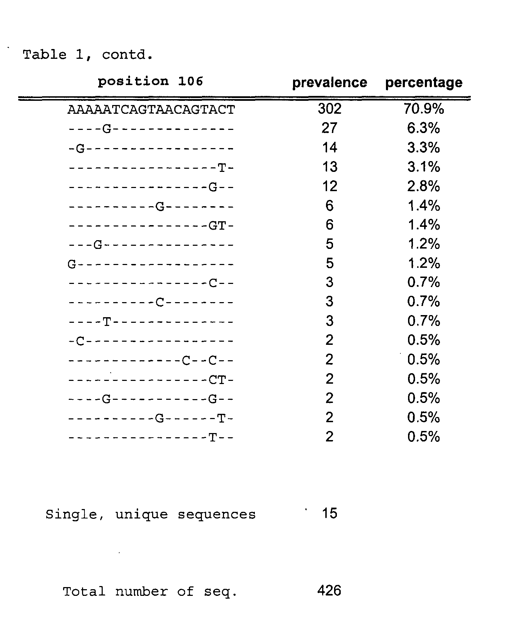

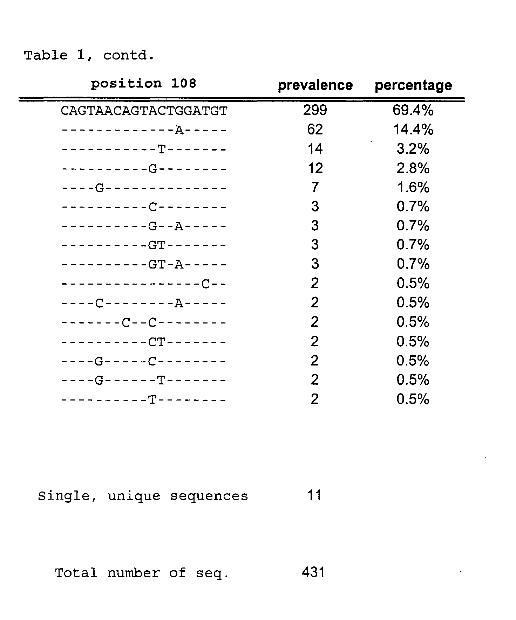

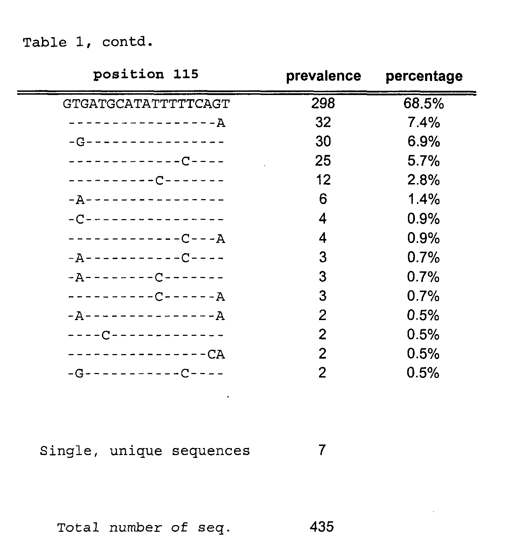

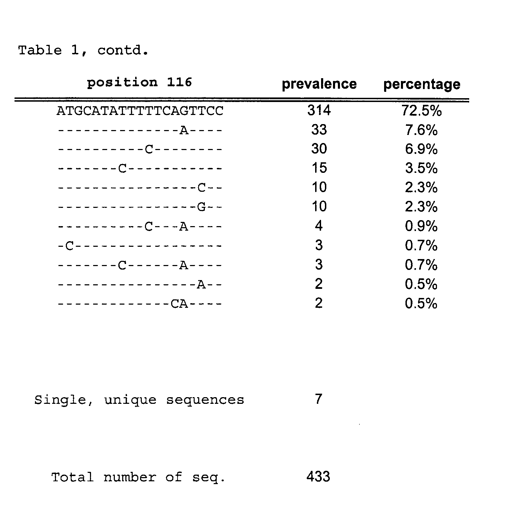

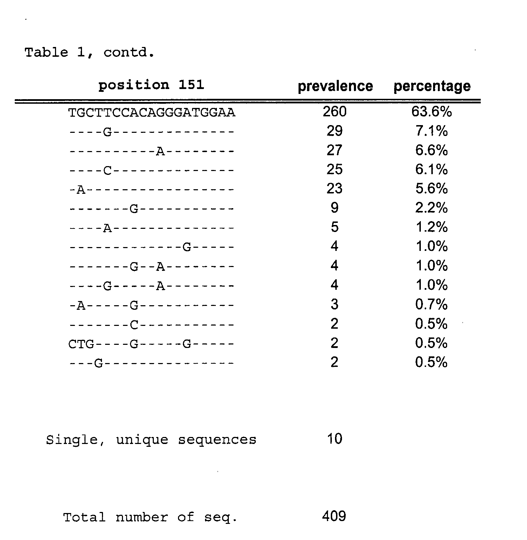

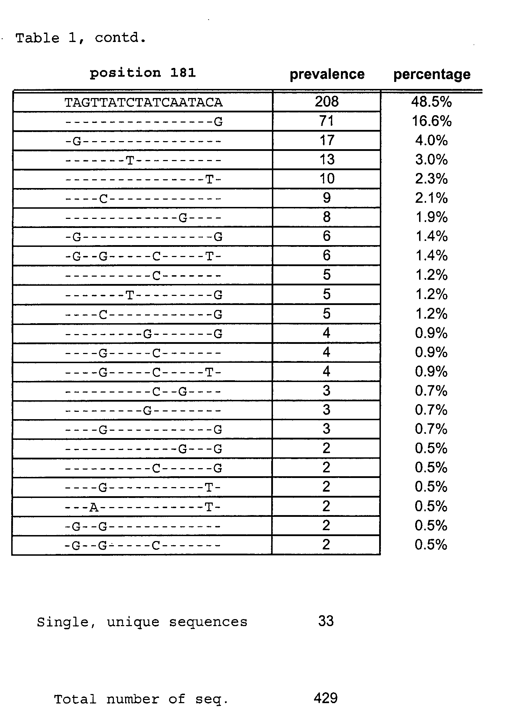

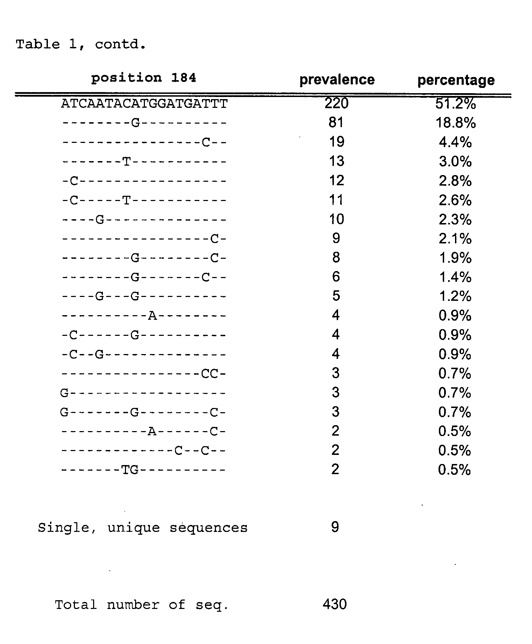

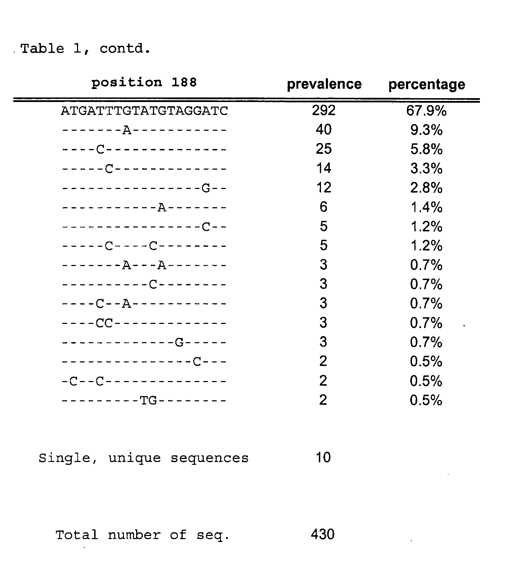

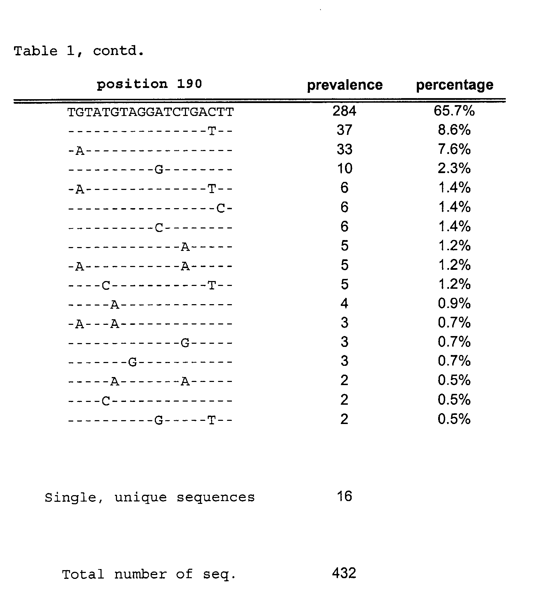

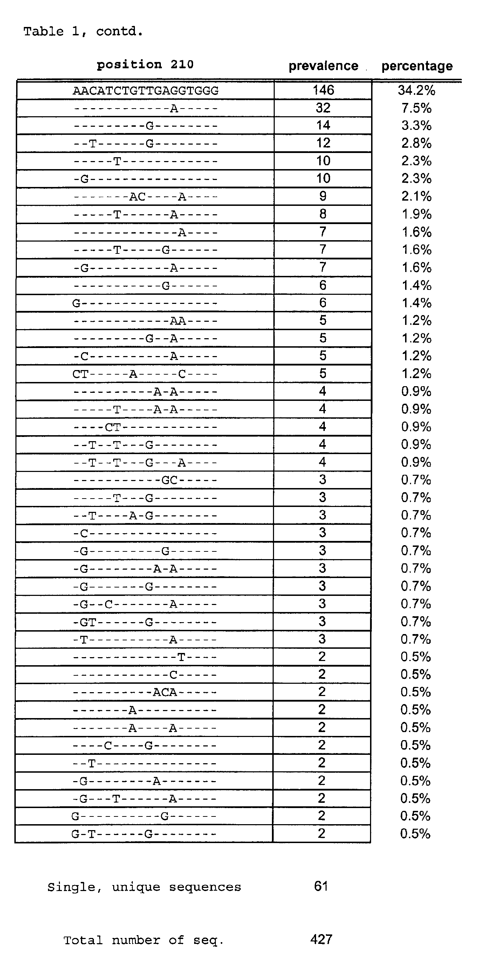

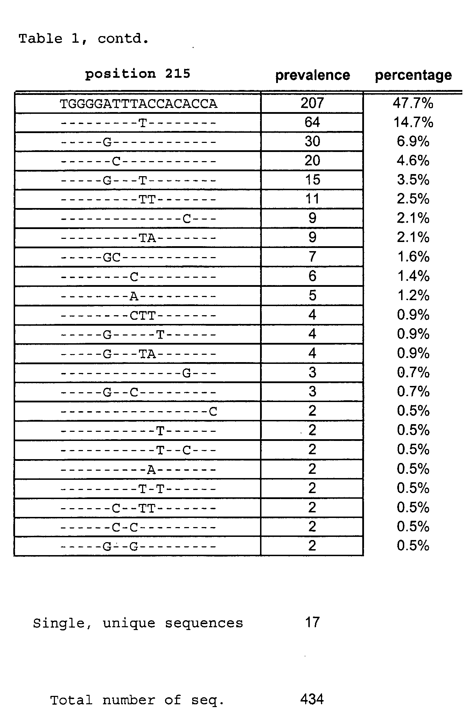

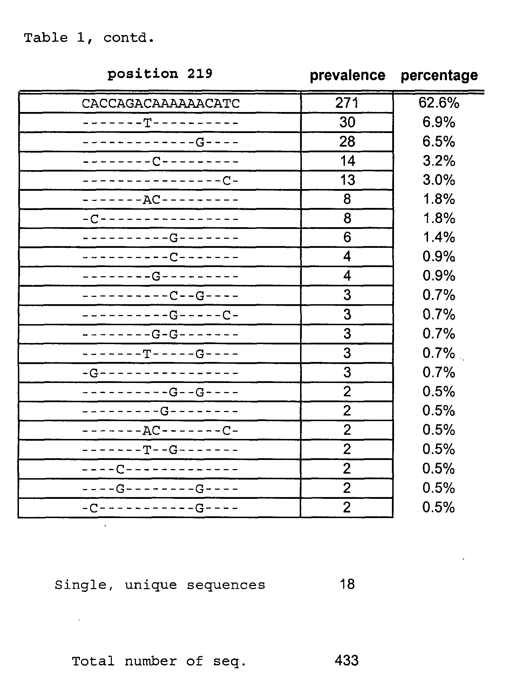

## Description

The invention relates to the field of molecular biology. In particular the invention relates to the field of molecular screening tools.

Many different methods for the typing nucleic acid have been devised. The currently most commonly used technique relies on probing of nucleic acid with a probe that is complementary to the nucleic acid of which the presence needs to be ascertained. Alternatively, an amplification reaction is performed that is specific for the nucleic acid of which the presence needs to be established.

A problem frequently encountered is that nucleic acids that are related to each other on the basis of nucleic acid sequence homology can not, or only with very special methods, be discriminated between. When such discrimination is needed, one typically relies on nucleic acid sequence analysis. However, for many purposes this is not convenient. For instance in a clinical setting a clinician needs the result of the typing as fast as possible to be able to adjust the treatment given to the patient on the basis of the results.

Thus, at least for some applications, alternatives for sequencing are sought that are quick, reliable and able to discriminate between homologous nucleic acids. Nucleic acid array technology is in principle capable of discriminating on the basis of sequence and methods for using such arrays can be made reliable and quick. Considering the number of nucleic acids that can be screened for in arrays such methods may also prove to be capable of discriminating between many different nucleic acids. However, the size of array is currently limited, resulting in arrays with only a limited number of test nucleic acids. Moreover, having very many different nucleic acids in the array is as yet very expensive. This is particularly so when nucleic acid needs to be typed of which many different forms can occur, such as for rapidly evolving nucleic acids. Typical examples of such rapidly evolving nucleic acids are pathogenic viruses, particularly RNA viruses, including retroviruses.

The present invention provides a very efficient method for generating arrays that are capable of detecting essentially all nucleic acids of relatively high significance from a family nucleic acids. The method is based on the surprising observation that although a sequence is highly variable, in nature only a limited number of possible permutations occur. It is not known what the mechanism for such restricted number of occurring permutations is. For instance for HIV, which is a highly variable virus, it has been observed that for a given region in the virus, the number of observed nucleic acids encoding said region is relatively small compared to number of nucleic acids that can potentially encode an amino acid sequence for the region. Given the redundancy of the genetic code one would expect a certain number of permutations. Given the tolerance of HIV for amino acid substitutions/insertions and deletions one would expect, on top of the number of permutations based on the genetic code, an additional number of possible permutations based on the mentioned tolerance of HIV. However, only a limited number of such possible permutations were actually observed in seropositive patients at a given point in time.

This striking observation, offers the possibility to limit the number of nucleic acids in the array. One only has to include the permutations actually occurring in nature, to be able to type HIV nucleic acid in a sample.

The invention is now as a non-limiting example exemplified using the family of nucleic acids coding for the various HIV strains. However, the invention is equally applicable to other families of nucleic acids.

Antiretroviral treatment of human immunodeficiency virus type 1 (HIV-1) infected individuals is based on inhibition of virus replication and is proven to increase both survival and quality of life of patients. In particular, antiretroviral drugs that inhibit virus replication by interacting with HIV-1 reverse transcriptase (RT), are commonly used. A standard method to evaluate the efficiency of antiviral therapy is monitoring of HIV-1 load in treated individuals by measuring HIV-1 RNA concentration in plasma or serum. HIV-1 level is one of decisive factors in AIDS progression. A general problem in antiretroviral treatment of HIV-1-infected individuals is the development of drug-resistant virus mutants. The rate of appearance of drug-resistant mutants and their susceptibility profiles to different drugs are highly variable in individual patients and depends on multiple factors, including therapy potency, drug levels, and patient compliance. Since presence of virus mutants resistant to antiretroviral drugs is resulting in therapy with these drugs being ineffective, it is important to monitor the appearance of these mutants. Their early detection in the patients allows choosing an alternative adequate therapy for them by changing drugs.

Current practice for monitoring the appearance of drug-resistant mutants is based on direct (without cloning) sequencing of the virus RT gene. HIV-1 resistance to antiretroviral drugs is associated with particular nucleotide substitutions, and their detection allows determining drug-resistant profiles of viruses. Sequencing of HIV-1 RT is an informative method, but it is expensive and laborious. This method requires each sequence to be individually edited, corrected, and finally authorized by a qualified physician. Moreover, HIV-1 populations in infected individuals are generally heterogeneous, and represent mixtures of different viruses, some of which could be drug-resistant while other are drug-susceptible. Although, in some cases, direct sequencing allows to detect both virus subpopulations, their discrimination in this method is poorly subjective, often not reliable, and could be done only based on visual analysis of each sequence position by a qualified physician. Ideally, sequencing dozens of individual clones could solve the latter problem, rather than whole virus populations, but this approach will considerably increase the cost of the analysis (more than 10 times).

A solution for these problems would be in developing microarrays in which HIV-1 RT sequence, including drug-resistant mutants, could be automatically determined based on results of hybridization of HIV-1 RT gene with various oligonucleotide probes. Diagnostic kits based on this approach are currently under development. An anticipated problem in their designing is related to high variation of HIV-1. An oligonucleotide probe should cover the codon of interest (in which drug-resistant mutation(s) could occur) as well as adjacent genetic regions (in total, about 20 nucleotides). Within these regions, there could be various mutations, which are essential to consider for determine virus sequence. Therefore, inclusion of just two oligonucleotides, corresponding to the wild-type and drug-resistant viruses, is not enough. This approach will result in sequence of many tested viruses being not determined (characterized an neither wild-type, nor drug-resistant).

Due to that, each drug-resistant position should be tested with more than two probes. Quantitative information on virus variation at and around positions of drug-resistant mutations and relative prevalence of particular mutants in human population is necessary for designing them.

For the optimal design of the oligonucleotide probe sets, we designed a method based on the analysis of HIV-1 sequence panel obtained from hundreds of patients, non-treated or treated with various anti-HIV-1 drugs. This panel is representative for HIV-1-infected individuals in Europe and North America, where HIV-1 subtype B is prevalent. An important aspect of one embodiment of the invention is defining the relative prevalence of various HIV-1 mutants (= oligonucleotide probes), starting from the most prevalent, than the second prevalent, etc., until to mutants which were found only in single human individual each. Based on this mutant distribution data, we were able to define the number and sequences of oligonucleotide probes which are necessary to detect the required proportion of various HIV-1 strains present in clinical patients. This proportion could be set to any number (i.e., 75%, 90%, 95%, etc.), and our data allow to determine, how many and which probes should be used in the assay to detect all these viruses.

Thus the invention provides a collection of binding moieties for a family of nucleic acids, which family comprises members of relative high and relative low significance, whereby said binding moieties are selected to be capable to identify, alone or in combination, essentially all members of said family of nucleic acids of relatively high significance. Nucleic acids of relative high significance can be nucleic acids encoding a particular mutation to be screened for. For instance, a mutation that is relevant for the prognosis and/or the treatment of a patient. Nucleic acids of relative high significance can also be nucleic acids that are observed with a particular frequency. Preferably, said nucleic acid of relative high significance comprises a particularly high frequency. Preferably, nucleic acids of relative high significance comprise a combination of the above, such as a particular mutation to be screened for which are observed with a particularly high frequency.

Preferably, said family of nucleic acids comprises members having nucleic acid sequence homology to each other. Although the invention was conceived studying HIV it is clear that the invention can also be used to design an array capable of typing nucleic acid from other sources. For instance HPV or other viruses. Another application of the invention is the design of a collection capable of typing nucleic acid in relation to an evolution tree. It is known that during evolution nucleic acid is conserved, but that it is subject to mutations, resulting in a family of nucleic acids having sequence homology. By determining the sequence of at least part of the nucleic acid of a number of species or sub-species one can generate an array capable of determining the evolutionary relationship of a test nucleic acid to the nucleic acids in the array.

Preferably, said family of nucleic acids comprises nucleic acids associated with a disease. In this case the family of nucleic acids consists of detected members of a pathogen causing the disease. Preferably, wherein said disease is a viral disease. Viruses are particularly prone to mutations. Specifically when the virus is an RNA virus or a retrovirus. Preferably, said nucleic acids of relative high significance are associated with a particular phenotype of said disease. Preferably, said phenotype comprises a drug resistant phenotype.

In one aspect of the invention, knowledge is obtained on the frequency with which a member of the family of nucleic acid is detected in a representative sample survey. This knowledge is subsequently used to determine whether said member comprises a relative high significance. By including a binding moiety to said member in the collection of binding moieties one is able to predict, based on the knowledge of the frequency, at least the lower confidence limit with which said collection is able to detect a member of said family of nucleic acids. Preferably, the frequency of more than one member is determined by representative sample survey. An additional advantage of knowing the frequency with which members of a family of nucleic acids occur in a sample survey is that that knowledge can be used to design the collection of binding moieties. For instance, inclusion of a binding moiety for a member, comprising a frequency of 40% in the sample survey, in the collection, results in a collection of binding moieties capable of identifying to at least 40% of the nucleic acids of said family in said sample survey. Inclusion of yet another binding moiety in the collection wherein said another moiety is capable of identifying a member of said family comprising a frequency of 20% in said sample survey results in a collection of binding moieties capable of identifying at least 60% of the nucleic acids of said family in said sample survey. Thus one can design collections of the invention such that they are capable of identifying members of said family with a predictable frequency in said sample survey. By appropriately designing the sample survey one can provide collections of the invention which are capable of identifying, with a predictable confidence limit, members of said family of nucleic acids. Members of relative high significance are in this aspect of the invention, members that together represent a certain frequency with which the members are found in a sample survey.

Although in a the preferred embodiment the collection of binding molecules comprises nucleic acid, the invention is not limited to nucleic acid arrays. Functional equivalents of nucleic acid such as peptide nucleic acid can be used as binding molecules. In principle, any molecule capable of binding to nucleic acid on the basis of the sequence of said nucleic acid is useful for designing the array and is considered to be a functional equivalent of nucleic acid. Alternatively, an antibody may be used as a binding molecule as long as said antibody is capable of binding to nucleic acid on the basis of the sequence of said nucleic acid.

The invention further provides a method for typing of nucleic acid in a sample comprising contacting said nucleic acid with a collection of binding moieties according to the invention and determining whether one or more binding moieties is bound to nucleic acid of said sample. Preferably, said sample is a clinical sample.

In another aspect the invention provides the use of a method of the invention for determining whether said sample comprises at least part of a member of relatively high significance of a family of nucleic acids. In yet another aspect the invention provides a use of a collection of binding molecules according to the invention, for diagnosing the severity of a disease.

The invention further provides a method for generating an array of binding molecules comprising the steps of
- analyzing a least part of the nucleic acid sequence of a number of members of a family of nucleic acid,
- determining nucleic acid sequences of relatively high significance and relatively low significance,
- selecting a set of binding molecules capable of binding to essentially all nucleic acid sequences of relatively high significance and,
- assembling said set into an array.

In yet another aspect the invention provides a method for diagnosing the severity of a disease of a patient, wherein said disease is caused by a pathogen containing a member of a family of nucleic acids, comprising contacting pathogen nucleic acid obtainable from said patient with a collection of binding molecules according to the invention and determining whether a binding molecule bound to sample nucleic acid.

### Examples

### Example 1

For analysis of mutations in the HIV-1 reverse transcriptase (RT) gene conferring resistance to antiviral treatment with therapeutics the following amino acid positions are considered: 41, 62, 65, 67, 70, 74, 75, 77, 98, 100, 103, 106, 108, 115, 116, 151, 181, 184, 188, 190, 210, 215, 219. In the following overviews the nucleic acid sequences variation around the codons of the above-mentioned amino acids is given. In each overview the codon itself and 8 nucleotides to the left and right are considered in the analysis. In each overview the most dominant sequence is given at the top, in all sequences below only the differences with the most dominant sequences are given (see table 1).

Because the distribution frequency of the sequences at each position is known, an array can be composed that has a sensitivity at certain position that is known due to knowledge about frequency. In this example enough sequences are taken (starting at the most dominant, than the second in line, third in line, etc. etc) to cover at least 75 % of the existing sequences at a particular position. For instance at amino acid position 219 this is achieved with 7 nucleotide sequences, for amino acid position 210 this is achieved with 40 nucleotide sequences. In this way an array with certain predicted performance can be composed. It is clear that arrays with less confidence at all or certain positions will require less sequences in the array.

The collection of sequences, or the complement thereof (i.e oligonucleotides) can be spotted on a filter membrane (for example nitrocellulose or ZetaProbe) on a known place (i.e. forming an array) using standard methods known by persons skilled in the art. Alternatively, the array may be spotted using state of the art spotting equipment enabling the depositiion of very small volumes which will result in extreme small arrays (micro chip format).

### Example 2

In this example nucleic acid was extracted from the serum (200 µl) of an HIV-1 infected individual using the "Boom" method (Boom R, Sol CJ, Salimans MM, Jansen CL, Wertheim-van Dillen PM, van der Noordaa J, 1990. Rapid and simple method for purification of nucleic acids. J Clin Microbiol; 28(3):495-503). After the extraction the isolated nucleic acid was stored at-20°C until used in subsequent experiments.

### Example 3

The RNA in the nucleic acid isolated in example 2 is reverse transcribed into cDNA using the following method. Mix in one reaction tube 3 µl 10x CMB buffer (100 mM Tris, pH = 8.3, 500 mM KCl, 1% triton), 0.2 µl dATP (25 mM solution), 0.2 µl dGTP (25 mM solution), 0.2 µl dTTP (25 mM solution), 0.2 µl dCTP (25 mM solution), 0.5 µl RNasin (40 units/µl), 4 µl primer 3' RT out (1 ng/µl, 5' TCTACTTGTCCATGCATGGCTTC), 1.7 µl water and 20 µl of the isolated nucleic acid. Vortex gently and leave at room temperature for 2 minutes to allow annealing. Subsequently add 1 µl 10x CMB buffer, 2.4 µl MgCl2 (100 mM solution), 0.5 µl MMLV-RT (from Life Technologies, 200 units/µl) and 6.1 µl water. Vortex gently and spin briefly to collect all liquids on the bottom of the tube, the total volume now is 40 µl. Incubate 2 hours at 37°C and subsequently 5 minutes at 95°C to inactivate the enzyme and spin briefly to collect all liquids on the bottom of the tube.

### Example 4

The newly made cDNA in example 3 is further amplified by PCR by adding to the 40 µl cDNA synthesis of example 3 the next solutions, 1.0 µl primer 5'PROT-I (100 ng/µl, 5' AGGCTAATTTTTTAGGGAAGATCTGGCCTTCC), 1.0 µl primer 3'ET21 (100 ng/µl, 5' AGCTGGCTACTATTTCTTTTGCTACTACAGGTGG), 6.0 µl 10x PCR buffer (500 mM Tris, pH = 8.3, 200 mM KCl, 1mg/ml BSA), 1.0 µl MgCl2 (100 mM solution), 0.05 µl dATP (25 mM solution), 0.05 µl dGTP (25 mM solution), 0.05 µl dTTP (25 mM solution), 0.05 µl dCTP (25 mM solution), 0.5 µl Taq DNA polymerase (5 units/µl) and 50.3 µl water. The tubes are "sealed" by the addition of two drops of paraffin oil and incubated in a 480 Perkin Elmer thermal cycler with the following protocol: 5 minutes 95°C, 35 cycles of 1 minute 95°C, 1 minute 55°C, 2 minutes 72°C followed by 10 minutes 72°C and lastly 10 minutes 4°C.

From this first PCR product the RT gene can be further amplified in a nested PCR with the following protocol, add to 5 µl of the first PCR reaction the following solutions: 1.0 µl primer RT19-new (100 ng/µl, 5' CACCTGTCAACATAATTGGAAG), 1.0 µl primer 3'RT22 (100 ng/µl, 5' AGGTTAAAATCACTAGCCATTGCTCTCC), 10.0 µl 10x PCR buffer (500 mM Tris, pH = 8.3, 200 mM KCl, 1mg/ml BSA), 2.0 µl MgCl2 (100 mM solution), 0.1 µl dATP (25 mM solution), 0.1 µl dGTP (25 mM solution), 0.1 µl dTTP (25 mM solution), 0.1 µl dCTP (25 mM solution), 1.0 µl ³²PαdATP, 0.4 µl Taq DNA polymerase (5 units/µl) and 79.2 µl water. The tubes are "sealed" by the addition of two drops of paraffin oil and incubated in a 480 Perkin Elmer thermal cycler with the following protocol: 10 minutes 95°C, 30 cycles of 1 minute 95°C, 1 minute 55°C, 2 minutes 72°C followed by 10 minutes 72°C. The amplified RT fragment in the nested PCR has a length of 1820 nucleotides.

The PCR product can be used directly for hybridization of the array (example 1) by applying methods well known to persons skilled in the art. For example: put the filter with the array in a hybridization botlle and add 10-15 ml hybridization mixture (20x SSC 250 ml, SDS [100 mM] 467 ml, NaPi [100 mM] 200 ml, 100xDenhardts 100 ml, fill up to 1 liter with water). Subsequently, add the labelled PCR product and hybridize in a hybridisation oven for at least 2 hours or overnight at 50°C.

Remove the hybridisation mixture and rinse the array twice with 3xSSC/1% SDS.

Subsequently, wash the array for 5-10 minutes with 3xSSC/1% SDS at 50°C.

Pack the array in household foil and expose to X-ray film. The intensity of the spots that can be detected on the autoradiograph indicate reaction of the HIV-1 sequences with the probes in the array. The probes that react positively indicate that that particular sequence is present in the HIV-1 genome that is analyzed and therefor indicate the presence of mutations conferring resistance.

### Example 5

The same PCR reactions (first and nested) as performed in example 4 (with omission of the ³²PαdATP in the reaction) can also be performed with identical primers that have to their 5' end an T7 promoter sequences attached:

5'PROT-I-T7 (5'
AATTCTAATACGACTCACTATAGGGAGGCTAATTTTTTAGGG
AAGATCTGGCCTTCC), 3'ET21-T7 (5' AATTCTAATACGACTCACTATAGGG
AGCTGGCTACTATTTCTTTTGCTACTACAGGTGG), RT19-new-T7 (5'
AATTCTAATACGACTCACTATAGGGCACCTGTCAACATAATTGGAAG),
3'RT22-T7 (5'
AATTCTAATACGACTCACTATAGGGAGGTTAAAATCACTAGCC
ATTGCTCTCC).

Alternatively, any combination of primers with and without the attached T7 promoter can be used in the PCR amplification. The PCR products that are obtained in such PCR reactions (see example 4 for details) can be subjected to transcription of the DNA into RNA using T7 RNA polymerase. This can be done according to standard transcription protocols known to persons skilled in the art, furthermore, several commercial products are currently available for performing in vitro transcription reactions (Promega, Life Technologies, Pharmacia, Ambion). Practically, during the in vitro transcription a labeled nucleotide is added to allow detection of the newly made RNA after retention on an array filter after hybridization. This label moiety can be a radioactive label, a fluorescent label or any other distinguishable group.

In case of the use of a radioactive label the in vitro generated RNA product can be used directly for hybridization of the array (example 1) by applying methods well known to persons skilled in the art. For example: put the filter with the array in a hybridization bottle and add 10-15 ml hybridization mixture (20x SSC 250 ml, SDS [100 mM] 467 ml, NaPi [100 mM] 200 ml, 100xDenhardts 100 ml, fill up to 1 liter with water). Subsequently, add the labeled in vitro generated RNA product and hybridize in a hybridization oven for at least 2 hours or overnight at 50°C.

Remove the hybridization mixture and rinse the array twice with 3xSSC/1% SDS.

Subsequently, wash the array for 5-10 minutes with 3xSSC/1% SDS at 50°C.

Pack the array in household foil and expose to X-ray film. The intensity of the spots that can be detected on the autoradiograph indicate reaction of the HIV-1 sequences with the probes in the array. The probes that react positively indicate that that particular sequence is present in the HIV-1 genome that is analyzed and therefor indicate the presence of mutations conferring resistance. In the current design of these examples (see example 1) the confidence per interrogated sequence is 75 %. The design of the array can be steered in such a way that this confidence can increased (more sequences necessary) or decreased (less sequences).

## Claims

1. A collection of binding moieties for a family of nucleic acids, which family comprises members of relative high and relative low significance, whereby said binding moieties are selected to be capable to identify, alone or in combination, essentially all members of said family of nucleic acids of relatively high significance.

2. A collection of binding moieties according to claim 1, wherein said family of nucleic acids comprises members having nucleic acid sequence homology to each other.

3. A collection of binding moieties according to claim 1 or claim 2, wherein said family of nucleic acids comprises nucleic acids associated with a disease.

4. A collection of binding moieties according to claim 3, wherein said disease is a viral disease.

5. A collection of binding moieties according to anyone of claims 1-4, wherein said nucleic acids of relative high significance are associated with a particular phenotype.

6. A collection of binding moieties according to claim 5, wherein said phenotype comprises a drug resistant phenotype.

7. A collection of binding moieties according to anyone of claims 1-6, wherein said binding moieties comprise nucleic acid and/or antigen binding parts of antibodies and/or derivatives thereof.

8. A method for typing of nucleic acid in a sample comprising contacting said nucleic acid with a collection of binding moieties according to anyone of claims 1-7 and determining whether one or more binding moieties bound to nucleic acid of said sample.

9. A method according to claim 8, wherein said sample is a clinical sample.

10. Use of a method according to claim 8 or claim 9, for determining whether said sample comprises at least part of a member of relatively high significance of a family of nucleic acids.
